# EUROPEAN PATENT SPECIFICATION

(11) **EP 3 174 855 B1**
(45) Date of publication and mention of the grant of the patent: **10.05.2023**
(21) Application number: 15826816.9
(22) Date of filing: 30.07.2015
(51) Int. Cl.: C07D 401/02

(54) **COFORMER SALTS OF (2S,3S)-METHYL 7-FLUORO-2-(4-FLUOROPHENYL)-3-(1-METHYL-1H-1,2,4-TRIAZOL-5-YL)-4-OXO-1,2,3,4-TETRAHYDROQUINOLINE-5-CARBOXYLATE AND METHODS OF PREPARING THEM**
COFORMER-SALZE VON (2S,3S)-METHYL-7-FLUOR-2-(4-FLUORPHENYL)-3-(1-METHYL-1H-1,2,4-TRIAZOL-5-YL)-4-OXO-1,2,3,4-TETRAHYDROCHINOLIN-5-CARBOXYLAT UND VERFAHREN ZUR HERSTELLUNG DAVON
SELS CONFORMÈRES DE 7-FLUORO-2-(4-FLUOROPHÉNYL)-3-(1-MÉTHYL-1H-1,2,4-TRIAZOL-5-YL)-4-OXO-1,2,3,4-TÉTRAHYDROQUINOLÉINE-5-CARBOXYLATE DE (2S,3S)-MÉTHYLE ET LEURS PROCÉDÉS DE PRÉPARATION

(30) Priority: 31.07.2014 US 201462031521 P
(43) Date of publication of application: 07.06.2017
(73) Proprietor: Medivation Technologies LLC, New York, NY 10001-2192 (US)
(72) Inventor: HENDERSON, Mark, Novato, CA 94949 (US); CAMPBELL, Colm, Novato, CA 94949 (US); JAGUSCH, Carsten, 90537 Feucht (DE); HERZ, Christian, Klaus, 90537 Feucht (DE); BAUER, Nico, 90537 Feucht (DE); BONNAUD, Thierry, Gillingham Dorset SP8 4XT (GB); LAMBERT, Olivier, SP8 4XT Gillingham, Dorset (GB)
(74) Representative: Pfizer
(86) International application number: PCT/US2015/042867
(87) International publication number: WO 2016/019125

(56) References cited:
- US-A- 4 415 504
- US-A1- 2011 027 226
- US-A1- 2011 196 153
- US-A1- 2011 196 153
- US-A1- 2011 301 350
- DIAZ ET AL.: 'Synthesis and Biological Evaluation of a New Series of Hexahydro-2H-pyrano[3,2- c]quinolines as Novel Selective s1 Receptor Ligands' J. MED. CHEM. vol. 56, no. 9, 01 January 2013, pages 3656 - 3665, XP055390546 DOI: 10.1021/JM400181K

## Description

### FIELD

This invention relates to a coformer salt of (2*S*,3*S*)-methyl 7-fluoro-2-(4-fluorophenyl)-3-(1-methyl-1*H*-1,2,4-triazol-5-yl)-4-oxo-1,2,3,4-tetrahydroquinoline-5-carboxylate optionally as a solvate and additionally optionally as a hydrate, including crystalline forms, and methods of preparing the (2*S*,3*S*)-methyl 7-fluoro-2-(4-fluorophenyl)-3-(1-methyl-1*H*-1,2,4-triazol-5-yl)-4-oxo-1,2,3,4-tetrahydroquinoline-5-carboxylate coformer salts.

### BACKGROUND

The compound (8*S*,9*R*)-5-fluoro-8-(4-fluorophenyl)-9-(1-methyl-1*H*-1,2,4-triazol-5-yl)-8,9-dihydro-2*H*-pyrido[4,3,2-de]phthalazin-3(7*H*)-one toluenesulfonate salt (Compound (A)) 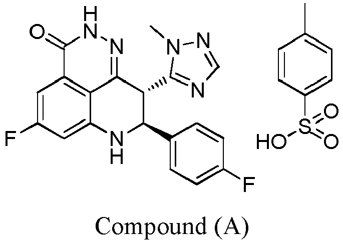 is an inhibitor of poly(ADP-ribose)polymerase (PARP). Methods of making it are described in WO2010017055, WO2011097602, and WO2012054698. However, the disclosed synthetic routes require chiral chromatography of one of the synthetic intermediates in the route to make Compound (A), methyl 7-fluoro-2-(4-fluorophenyl)-3-(1-methyl-1*H*-1,2,4-triazol-5-yl)-4-oxo-1,2,3,4-tetrahydroquinoline-5-carboxylate (Intermediate (A)), 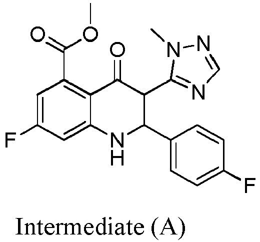 to yield the chirally pure (2*S*,3*S*)-methyl 7-fluoro-2-(4-fluorophenyl)-3-(1-methyl-1*H*-1,2,4-triazol-5-yl)-4-oxo-1,2,3,4-tetrahydroquinoline-5-carboxylate (Compound (1))

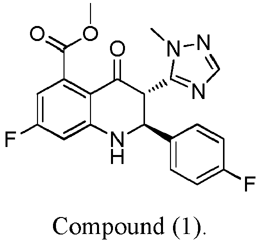

Using conventional chiral chromatography is often solvent and time intensive. Use of more efficient chromatography methods, such as simulated moving bed (SMB) chromatography still requires the use of expensive chiral chromatography resins, and is not practical on a large scale to purify pharmaceutical compounds. Also, maintaining Compound (1) in solution for an extended time period during chromatography can lead to epimerization at the 9-position and cleavage of the methyl ester group in Compound (1). Replacing the chromatography step with crystallization step(s) to purify Compound (1) is desirable and overcomes these issues. Therefore, it is desirable to find an alternative to the use of chiral chromatography separations to obtain enantiomeric Compound (1).

Disclosed herein are coformer salts of (2*S*,3*S*)-methyl 7-fluoro-2-(4-fluorophenyl)-3-(1-methyl-1*H*-1,2,4-triazol-5-yl)-4-oxo-1,2,3,4-tetrahydroquinoline-5-carboxylate and methods of preparing them, which solve the described difficulties.

The embodiments described herein can lead to significant increases in the purity of the desired compounds and can confer added advantages in manufacturing Compound (A) for regulatory approval and marketing. The embodiments described herein allow for a more consistent production of the compounds that meet the regulatory authorities' standards and guidelines for purity for an approved drug product. An appreciable reduction in manufacturing time and expense can also be achieved. A significant reduction of the "cis/trans" isomeric impurities of Compound (1) (where the cis isomers are the (2*R*, 3*S*) and *(2S,* 3*R*) forms, and the trans isomer is the (2*R*, 3*R*) form) can be achieved. A high degree of enantiomeric selectivity of Compound (1) can be achieved.

### BRIEF DESCRIPTION OF THE DRAWINGS

Figure 1. depicts the XRPD for Compound (1a), Step 1a for Examples 1 and 3 obtained using XRPD Procedure 2.
Figures 2a. and 2b. depict the chiral HPLC of Compound (1a), Step 1a in Example 3.
Figures 3. depicts the ¹H NMR for Compound (1a), Step 1a in Example 3.
Figure 4. depicts the TGA/DSC of Compound (1a), Step 1a in Example 3.
Figure 5. depicts the XRPD for Compound (1a), Step 1b in Example 3 (top) and Compound (1a) from Example 1 obtained using XRPD Procedure 2.
Figure 6. depicts the chiral HPLC for Compound (1a), Step 1b in Example 3.
Figure 7. depicts the XRPD for Compound (1) in Example 3, Step 2 and Intermediate (A).
Figure 8. depicts the ¹H NMR for Compound (1) in Example 3 and Intermediate (A).
Figure 9. depicts the XRPDs for Compound (1b) in Example 5, Compound (1b) from Example 1, and Intermediate (A) obtained using XRPD Procedure 2.
Figure 10. depicts the chiral HPLC for Compound (1b) in Example 5.
Figure 11. 1H NMR for Compound (1b) in Example 5.
Figure 12a. depicts the TGA and DSC for Compound (1b) in Example 5.
Figure 12b. depicts the DSC for Compound (1b) in Example 5 (bottom) and Compound (1b) in Example 1.
Figure 13a. depicts the ¹H NMR (in DMSO-d₆) for Compound (1a) in Example 4.
Figure 13b. depicts the ¹³C NMR (in DMSO-d₆) for Compound (1a) in Example 4.
Figure 14. depicts the IR spectrum for Compound (1a) in Example 4.
Figure 15. depicts the DSC for Compound (1a) in Example 4.
Figure 16. depicts the chiral HPLC for Compound (1a) in Example 4.

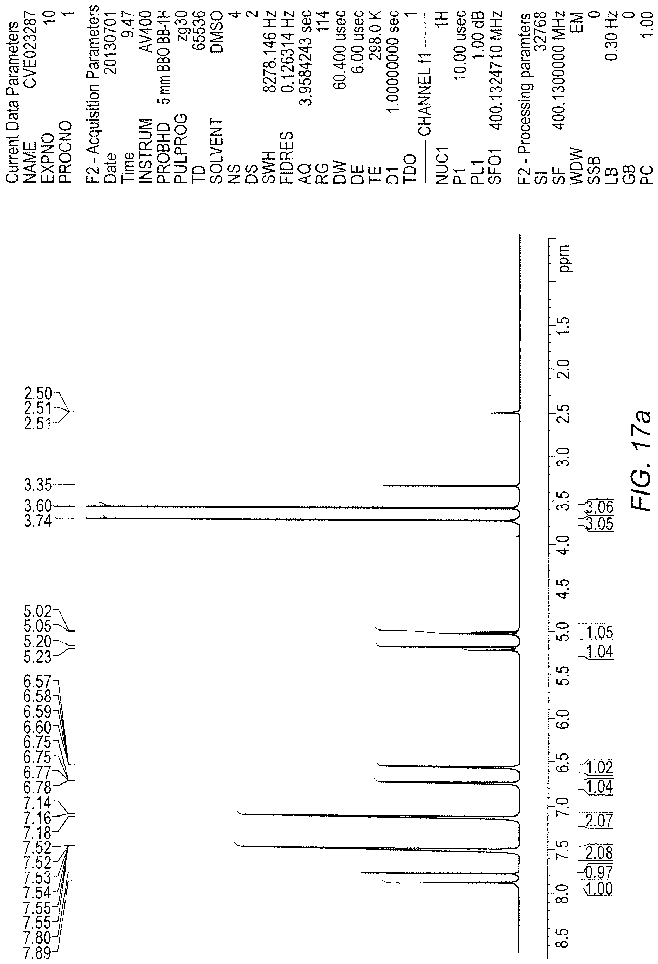

Figure 17a. depicts the ¹H NMR (in DMSO-d₆) for Compound (1) in Example 4.

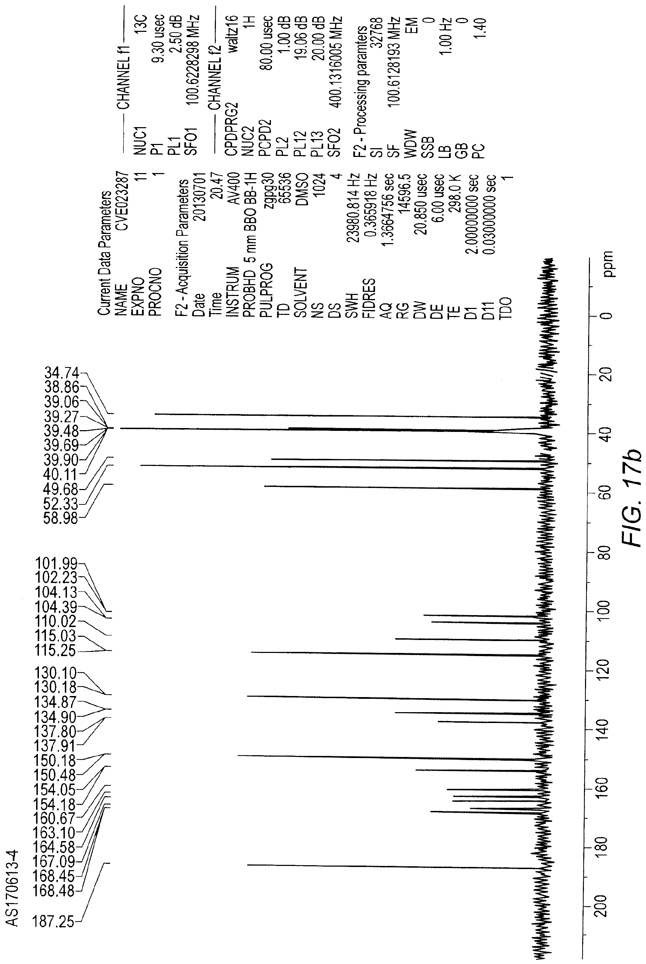

Figure 17b. depicts the ¹³C NMR (in DMSO-d₆) for Compound (1) in Example 4.

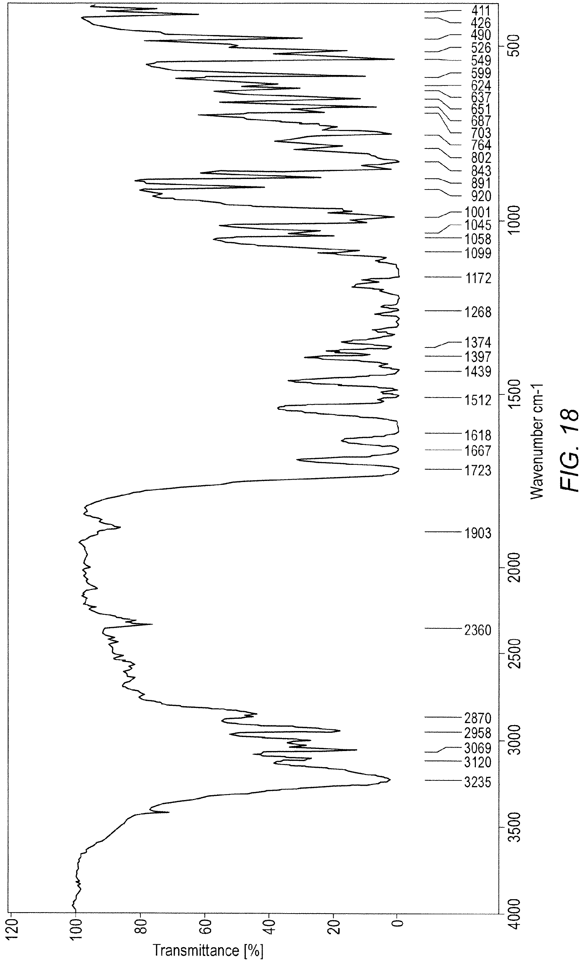

Figure 18. depicts the IR spectrum for Compound (1) in Example 4.
Figure 19. depicts the DSC for Compound (1) in Example 4.
Figure 20. depicts the chiral HPLC for Compound (1) in Example 4.

### SUMMARY OF THE INVENTION

In one aspect, provided herein is a coformer salt of (2*S*,3*S*)-methyl 7-fluoro-2-(4-fluorophenyl)-3-(1-methyl-1*H*-1,2,4-triazol-5-yl)-4-oxo-1,2,3,4-tetrahydroquinoline-5-carboxylate optionally as a solvate and additionally optionally as a hydrate thereof, which is [(1S)-endo]-(+)-3-bromo-10-camphor sulfonic acid salt of (2S,3S)-methyl 7-fluoro-2-(4-fluorophenyl)-3-(1-methyl-1H-1,2,4-triazol-5-yl)-4-oxo-1,2,3,4-tetrahydroquinoline-5-carboxylate and which is a crystalline form exhibiting a solid state ¹³C NMR spectrum with peaks at 210.3, 25.3, 21.8, 20.8, 19.5, and 18.5 ppm ± 0.2 ppm referenced to residual peaks from DMSO-d₆.

In another aspect provided herein is a method of preparing the coformer salt of (2*S*,3*S*)-methyl 7-fluoro-2-(4-fluorophenyl)-3-(1-methyl-1*H*-1,2,4-triazol-5-yl)-4-oxo-1,2,3,4-tetrahydroquinoline-5-carboxylate comprising (1) treating methyl 7-fluoro-2-(4-fluorophenyl)-3-(1-methyl-1*H*-1,2,4-triazol-5-yl)-4-oxo-1,2,3,4-tetrahydroquinoline-5-carboxylate with a coformer in one or more step 1a) solvent(s) selected from MIBK, MEK, ethanol, and water at an elevated temperature to form a step 1a) solution; (2) allowing the step 1a) solution to stand under conditions sufficient to precipitate the coformer salt in a crystalline form; and (3) isolating the coformer salt in the crystalline form.

In certain embodiments, the coformer salt is a [(1*S*-*endo*]-(+)-3-bromo-10-camphor sulfonate of Compound (1) and the step 1a) solvents are selected from acetone, methylethylketone, methylisobutylketone (MIBK), methanol, ethanol, propanol, isopropanol, and butanol.

In certain embodiments, the coformer salt is a [(1*S*-*endo*]-(+)-3-bromo-10-camphor sulfonate of Compound (1) and the step 1a) solvents are MIBK, water, and ethanol.

In certain embodiments, the coformer salt is a [(1*S*-*endo*]-(+)-3-bromo-10-camphor sulfonate of Compound (1) and the step 1a) solvents are MIBK and ethanol.

In certain embodiments, the method further comprises recrystallizing or reslurrying the coformer salt in one or more step 1b) solvent(s).

In certain embodiments, the coformer salt of (2*S*,3*S*)-methyl 7-fluoro-2-(4-fluorophenyl)-3-(1-methyl-1*H*-1,2,4-triazol-5-yl)-4-oxo-1,2,3,4-tetrahydroquinoline-5-carboxylate is in crystalline form after recrystallizing or reslurrying in step 1b) solvent(s).

In certain embodiments, the method further comprises suspending the coformer salt of (2*S*,3*S*)-methyl 7-fluoro-2-(4-fluorophenyl)-3-(1-methyl-1*H*-1,2,4-triazol-5-yl)-4-oxo-1,2,3,4-tetrahydroquinoline-5-carboxylate in one or more step 2a) solvent(s) selected from water, acetone, IPA, or methanol at room temperature or elevated temperature to form a step 2a) solution and treating the step 2a) solution with a base selected from NaOH, NH₃ (optionally 25% aqueous NH₃), NaCO₃, NaOAc, or NaHCO₃; allowing the step 2a) solution to stand under conditions sufficient to precipitate a crystalline form of the (2*S*,3*S*)-methyl 7-fluoro-2-(4-fluorophenyl)-3-(1-methyl-1*H*-1,2,4-triazol-5-yl)-4-oxo-1,2,3,4-tetrahydroquinoline-5-carboxylate; and isolating a crystalline form of (2*S*,3*S*)-methyl 7-fluoro-2-(4-fluorophenyl)-3-(1-methyl-1*H*-1,2,4-triazol-5-yl)-4-oxo-1,2,3,4-tetrahydroquinoline-5-carboxylate.

In certain embodiments, the step 2a) solvents are selected from acetone, methylethylketone, methylisobutylketone, methanol, ethanol, propanol, or isopropanol; and the base is aqueous NH₃.

In certain embodiments, the step 2a) solvents are acetone, methanol, and isopropanol; and the base is aqueous NH₃.

In certain embodiments, the method further comprises recrystallizing or reslurrying the (2*S*,3*S*)-methyl 7-fluoro-2-(4-fluorophenyl)-3-(1-methyl-1*H*-1,2,4-triazol-5-yl)-4-oxo-1,2,3,4-tetrahydroquinoline-5-carboxylate in one or more step 2b) solvent(s).

In another aspect, provided herein is a compound (2*S*,3*S*)-methyl 7-fluoro-2-(4-fluorophenyl)-3-(1-methyl-1*H*-1,2,4-triazol-5-yl)-4-oxo-1,2,3,4-tetrahydroquinoline-5-carboxylate optionally as a solvate and additionally optionally as a hydrate prepared by treating a coformer salt of (2*S*,3*S*)-methyl 7-fluoro-2-(4-fluorophenyl)-3-(1-methyl-1*H*-1,2,4-triazol-5-yl)-4-oxo-1,2,3,4-tetrahydroquinoline-5-carboxylate with a base and isolating the (2*S*,3*S*)-methyl 7-fluoro-2-(4-fluorophenyl)-3-(1-methyl-1*H*-1,2,4-triazol-5-yl)-4-oxo-1,2,3,4-tetrahydroquinoline-5-carboxylate.

### DETAILED DESCRIPTION

**Abbreviations**

| **Abbreviation** | **Meaning** |
|---|---|
| ACN | acetonitrile |
| DCM | dichloromethane |
| DMF | N,N-dimethylformamide |
| DSC | differential scanning calorimetry |
| EA | ethyl acetate |
| e.e. | enantiomeric excess |
| EtOH | ethanol |
| equiv | equivalent |
| g | gram |
| IPA | isopropanol |
| IR | infrared |
| mHz | megaHertz |
| MEK | methylethylketone |
| MIBK | methylisobutylketone |
| mL | milliliter |
| mol | mole |
| NaOH | sodium hydroxide |
| NMR | nuclear magnetic resonance |
| TGA | thermogravimetric analysis |
| THF | tetrahydrofuran |
| XRPD | X-ray powder diffraction |

### Definitions

To facilitate understanding of the disclosure set forth herein, a number of terms are defined below. Generally, the nomenclature used herein and the laboratory procedures in organic chemistry, medicinal chemistry, and pharmacology described herein are those well-known and commonly employed in the art. Unless defined otherwise, all technical and scientific terms used herein generally have the same meaning as commonly understood by one of ordinary skill in the art to which this disclosure belongs. In the event that there is a plurality of definitions for a term used herein, those in this section prevail unless stated otherwise.

As used throughout this application and the appended claims, the following terms have the following meanings:

As used herein, the singular forms "a", "an" and "the" include plural referents unless the content clearly dictates otherwise. Thus, for example, reference to "a compound" includes a mixture of two or more compounds, and the like.

As used herein, and unless otherwise specified, the terms "about" and "approximately," when used in connection with doses, amounts, or weight percent of ingredients of a composition or a dosage form, mean a dose, amount, or weight percent that is recognized by those of ordinary skill in the art to provide a pharmacological effect equivalent to that obtained from the specified dose, amount, or weight percent. In certain embodiments, the terms "about" and "approximately," when used in this context, contemplate a dose, amount, or weight percent within 15%, within 10%, within 5%, within 4%, within 3%, within 2%, within 1%, or within 0.5% of the specified dose, amount, or weight percent.

As used herein, and unless otherwise specified, the terms "about" and "approximately," when used in connection with a numeric value or range of values which is provided to describe a particular solid form, *e*.*g*., a specific temperature or temperature range, such as, for example, that describing a melting, dehydration, desolvation or glass transition; a mass change, such as, for example, a mass change as a function of temperature or humidity; a solvent or water content, in terms of, for example, mass or a percentage; or a peak position, such as, for example, in analysis by, for example, ¹³C NMR, DSC, TGA and XRPD; indicate that the value or range of values may deviate to an extent deemed reasonable to one of ordinary skill in the art while still describing the particular solid form. In certain embodiments, the terms "about" and "approximately," when used in this context, indicate that the numeric value or range of values may vary by 5%, 4%, 3%, 2%, 1%, 0.9%, 0.8%, 0.7%, 0.6%, 0.5%, 0.4%, 0.3%, 0.2% or 0.1% of the recited value or range of values while still describing the particular solid form.

The term "amorphous" or "amorphous form" is intended to mean that the substance, component, or product in question is not substantially crystalline as determined, for instance, by XRPD or where the substance, component, or product in question, for example is not birefringent when viewed microscopically. In certain embodiments, a sample comprising an amorphous form of a substance may be substantially free of other amorphous forms and/or crystalline forms.

The term "crystalline form" or "crystal form" refers to a crystalline solid form of a chemical compound, including, but not limited to, a single-component or multiplecomponent crystal form, *e*.*g*., a polymorph of a compound; or a solvate, a hydrate, a clathrate, a cocrystal, a salt of a compound, or a polymorph thereof. The term "crystal forms" and related terms herein refers to the various crystalline modifications of a given substance, including, but not limited to, polymorphs, solvates, hydrates, co-crystals and other molecular complexes, as well as salts, solvates of salts, hydrates of salts, other molecular complexes of salts, and polymorphs thereof. Crystal forms of a substance can be obtained by a number of methods, as known in the art. Such methods include, but are not limited to, melt recrystallization, melt cooling, solvent recrystallization, recrystallization in confined spaces such as, *e*.*g*., in nanopores or capillaries, recrystallization on surfaces or templates such as, *e.g*., on polymers, recrystallization in the presence of additives, such as, *e*.*g*., co-crystal counter-molecules, desolvation, dehydration, rapid evaporation, rapid cooling, slow cooling, vapor diffusion, sublimation, grinding and solvent-drop grinding.

Techniques for characterizing crystal forms and amorphous forms include, but are not limited to, TGA, DSC, XRPD, single crystal X-ray diffractometry, vibrational spectroscopy, *e*.*g*., IR and Raman spectroscopy, solid-state NMR, optical microscopy, hot stage optical microscopy, SEM, electron crystallography and quantitative analysis, PSA, surface area analysis, solubility studies and dissolution studies.

As used herein and unless otherwise indicated, the term "hydrate" means a compound or salt thereof, further including a stoichiometric or non-stoichiometric amount of water bound by non-covalent intermolecular forces.

As used herein and unless otherwise indicated, the term "solvate" means a solvate formed from the association of one or more solvent molecules to a compound provided herein or salt thereof. The term "solvate" includes hydrates (*e*.*g*., hemihydrates, monohydrate, dihydrate, trihydrate, tetrahydrate, and the like).

The term "polymorph" or "polymorphic form" refers to one of two or more crystal forms that comprise the same molecule, molecules or ions. Different polymorphs may have different physical properties such as, for example, melting temperatures, heats of fusion, solubilities, dissolution rates, and/or vibrational spectra as a result of the arrangement or conformation of the molecules or ions in the crystal lattice. The differences in physical properties exhibited by polymorphs may affect pharmaceutical parameters, such as storage stability, compressibility, density (important in formulation and product manufacturing), and dissolution rate (an important factor in bioavailability). Differences in stability can result from changes in chemical reactivity (*e*.*g*., differential oxidation, such that a dosage form discolors more rapidly when comprised of one polymorph than when comprised of another polymorph), mechanical changes (*e.g*., tablets crumble on storage as a kinetically favored polymorph converts to thermodynamically more stable polymorph), or both (*e*.*g*., tablets of one polymorph are more susceptible to breakdown at high humidity). As a result of solubility/dissolution differences, in the extreme case, some polymorphic transitions may result in lack of potency or, at the other extreme, toxicity. In addition, the physical properties of a crystalline form may be important in processing; for example, one polymorph might be more likely to form solvates or might be difficult to filter and wash free of impurities (*e*.*g*., particle shape and size distribution might be different between polymorphs).

As used herein, "substantially pure" refers to a substance or mixture that is substantially free of other compounds, stereoisomers, coformer salts, solvates, hydrates, or other solid forms thereof, including other crystalline or amorphous forms. In certain contexts, a "substantially pure" compound, such as substantially pure (2*S*,3*S*)-methyl 7-fluoro-2-(4-fluorophenyl)-3-(1-methyl-1*H*-1,2,4-triazol-5-yl)-4-oxo-1,2,3,4-tetrahydroquinoline-5-carboxylate or a coformer salt or solvate thereof, can mean substantially free of other chemical compounds, for example, unreacted precursors and side products that might be present in process for preparing the desired compound. In other contexts, as used herein, a "substantially pure" solid form (*e*.*g*., crystalline form or amorphous form) of (2*S*,3*S*)-methyl 7-fluoro-2-(4-fluorophenyl)-3-(1-methyl-1*H*-1,2,4-triazol-5-yl)-4-oxo-1,2,3,4-tetrahydroquinoline-5-carboxylate or a salt or solvate thereof can mean substantially free of other solid forms of (2*S*,3*S*)-methyl 7-fluoro-2-(4-fluorophenyl)-3-(1-methyl-1*H*-1,2,4-triazol-5-yl)-4-oxo-1,2,3,4-tetrahydroquinoline-5-carboxylate or salts or solvates thereof. In certain contexts, "stereomerically pure" means a composition that comprises one stereoisomer of a compound and is substantially free of other stereoisomers of that compound.

As used herein the term "vol" or "vols" means a weight/volume ratio of solid reactants to liquid solvents. For example, 250 g of a solid substance in 10 vols of a solvent means the substance is dissolved in 10 × 250 mL, or 2.5 L, of solvent.

It will be understood that a coformer salt of (2*S*,3*S*)-methyl 7-fluoro-2-(4-fluorophenyl)-3-(1-methyl-1*H*-1,2,4-triazol-5-yl)-4-oxo-1,2,3,4-tetrahydroquinoline-5-carboxylate comprises a cation of (2*S*,3*S*)-methyl 7-fluoro-2-(4-fluorophenyl)-3-(1-methyl-1*H*-1,2,4-triazol-5-yl)-4-oxo-1,2,3,4-tetrahydroquinoline-5-carboxylate (*e*.*g*., in one embodiment, protonated at one atomic position, or in other embodiments, protonated at more than one atomic position) and an anion of the coformer acid.

### Embodiments

In one aspect, this disclosure provides a coformer salt of (2*S*,3*S*)-methyl 7-fluoro-2-(4-fluorophenyl)-3-(1-methyl-1*H*-1,2,4-triazol-5-yl)-4-oxo-1,2,3,4-tetrahydroquinoline-5-carboxylate (hereinafter referred to as "coformer salt of Compound (1)") which is a [(1*S*)-*endo*]-(+)-3-bromo-10-camphor sulfonic acid salt of (2*S*,3*S*)-methyl 7-fluoro-2-(4-fluorophenyl)-3-(1-methyl-1*H*-1,2,4-triazol-5-yl)-4-oxo-1,2,3,4-tetrahydroquinoline-5-carboxylate (the coformer salt hereinafter referred to as "Compound (1a)") optionally as a solvate and additionally optionally as a hydrate thereof. Compound (1a) may comprise a cation to anion molar ratio of about 1:1. The cation to anion molar ratio may be about 1:1.1, about 1:1.15, about 1:1.2, or about 1:1.3.

In certain embodiments, Compound (1a) is unsolvated.

In certain embodiments, Compound (1a) is a solvate. In certain embodiments, the solvate form is a hydrate thereof. The solvate form may be an ethanolate solvate. Alternatively, the solvate form may be an ethanolate solvate and hydrate. The ratio of Compound (1a) to the ethanol solvate may be about 1:0.4, about 1:0.5, about 1:0.6, or about 1:0.7. The ratio of Compound (1a) to the hydrate may be about 1:0.4, about 1:0.5, about 1:0.6, or about 1:0.7.

Compound (1a), and the solvates and hydrates thereof may be stereochemically pure.

The substantially pure coformer salt may comprise substantially pure (2*S*,3*S*)-methyl 7-fluoro-2-(4-fluorophenyl)-3-(1-methyl-1*H*-1,2,4-triazol-5-yl)-4-oxo-1,2,3,4-tetrahydroquinoline-5-carboxylate that is substantially free of other stereoisomers including, for example, (2*R*,3*R*)-methyl 7-fluoro-2-(4-fluorophenyl)-3-(1-methyl-1*H*-1,2,4-triazol-5-yl)-4-oxo-1,2,3,4-tetrahydroquinoline-5-carboxylate, (2*S*,3*R*)-methyl 7-fluoro-2-(4-fluorophenyl)-3-(1-methyl-1*H*-1,2,4-triazol-5-yl)-4-oxo-1,2,3,4-tetrahydroquinoline-5-carboxylate, and (2*S*,3*S*)-methyl 7-fluoro-2-(4-fluorophenyl)-3-(1-methyl-1*H*-1,2,4-triazol-5-yl)-4-oxo-1,2,3,4-tetrahydroquinoline-5-carboxylate. Compound (1a) may comprise approximately 100% by weight of the specific stereoisomer of Compound (1), wherein the percentage is based on the total amount of combined stereoisomers in the stereochemically pure coformer salt.

Compound (1a), and the solvates and hydrates thereof may comprise greater than about 80 percent by weight of Compound (1) and less than about 20 percent by weight of any stereoisomers of Compound (1), greater than about 90 percent by weight of Compound (1) and less than about 10 percent by weight of any stereoisomers of Compound (1), greater than about 95 percent by weight of Compound (1) and less than about 5 percent by weight of any stereoisomers of Compound (1), greater than about 97 percent by weight of Compound (1) and less than about 3 percent by weight of any stereoisomers of Compound (1), greater than about 99 percent by weight of Compound (1) and less than about 1 percent by weight of any stereoisomers of Compound (1), or greater than about 99.5 percent by weight of Compound (1) and less than about 0.5 percent by weight of any stereoisomers of Compound (1). The above percentages are based on the total amount of combined stereoisomers in stereochemically pure coformer salt.

Compound (1a), and the solvates and hydrates thereof is substantially free of one or more other particular crystal forms, amorphous forms, and/or other chemical compounds. In particular, Compound (1a), and the solvates and hydrates thereof comprises less than about 10%, less than about 5%, less than about 3%, less than about 2%, less than about 1%, less than about 0.75%, less than about 0.5%, less than about 0.25%, or less than about 0.1% by weight of one or more other crystal forms or amorphous forms of (2*S*,3*S*)-methyl 7-fluoro-2-(4-fluorophenyl)-3-(1-methyl-1*H*-1,2,4-triazol-5-yl)-4-oxo-1,2,3,4-tetrahydroquinoline-5-carboxylate and/or other chemical compounds that may result from the synthetic processes disclosed herein. In certain embodiments, Compound (1a) is substantially free of an amorphous form.

In certain embodiments, Compound (1a), and the solvates and hydrates thereof, has a crystalline salt purity which is of at least about 90%, at least about 95%, at least about 97%, at least about 98%, at least about 99%, at least about 99.2%, at least about 99.5%, at least about 99.6%, at least about 99.7% or at least about 99.8% by weight of a single crystalline form, with the remainder of the total weight which may be other crystalline or amorphous forms and/or other compounds.

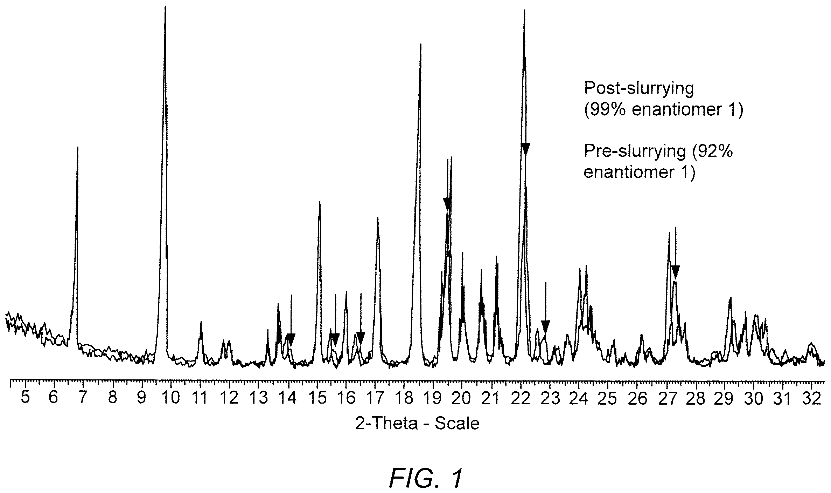

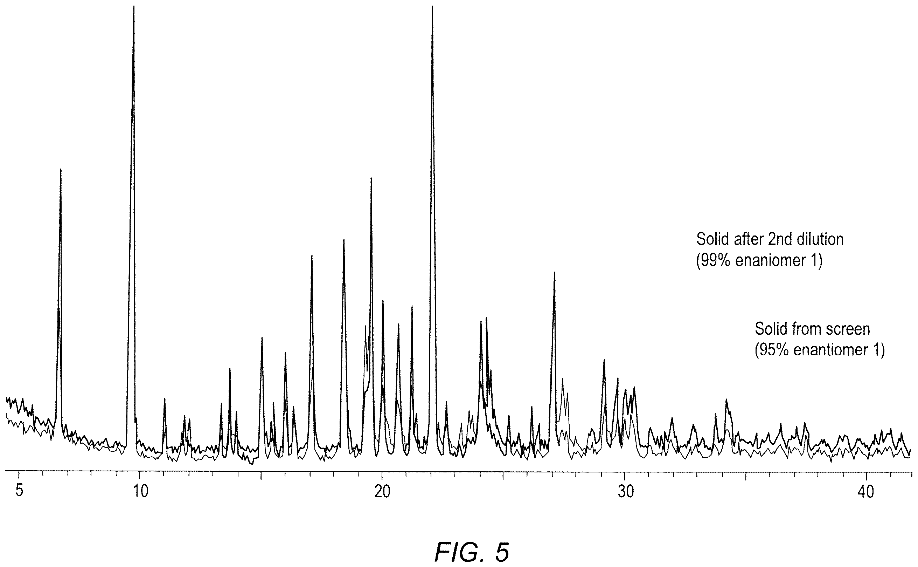

The XRPD pattern of crystalline Compound (1a) comprises one or more (*e*.*g*., one, two, three, four, five, or at least two, at least three, or at least four) characteristic peaks selected from peaks with 2θ angle degrees ± 0.2 2θ of about 6.7, 9.7, 18.5, 19.5, and 22. In certain embodiments, the XRPD pattern of crystalline Compound (1a) comprises a characteristic peak selected from peaks with 2θ angle degrees ± 0.2 2θ of about 6.7 and 9.7. In certain embodiments, the XRPD pattern of crystalline Compound (1a) is substantially as provided in Figures 1 or 5.

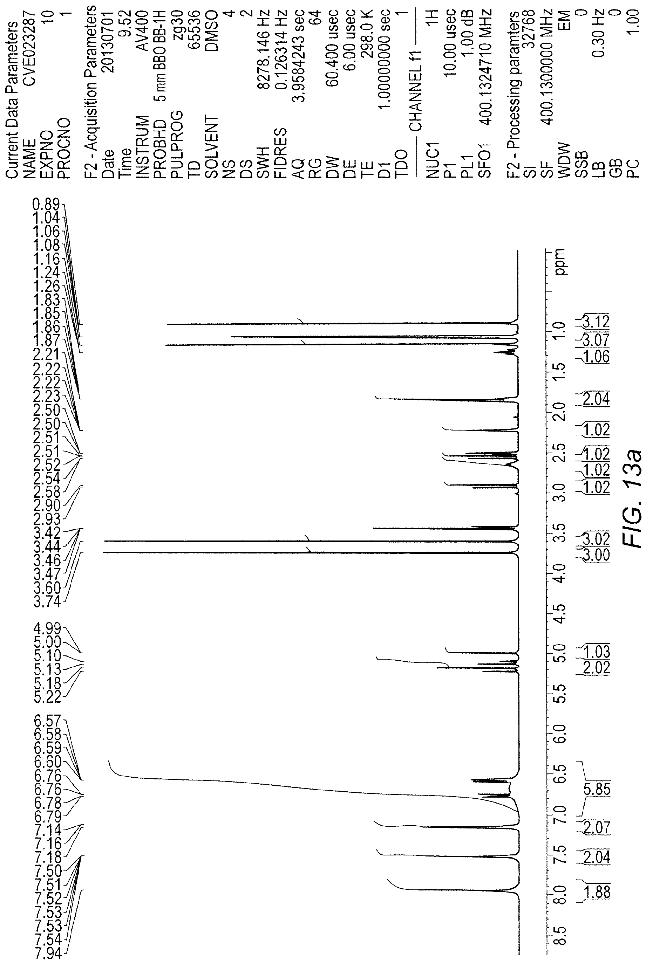

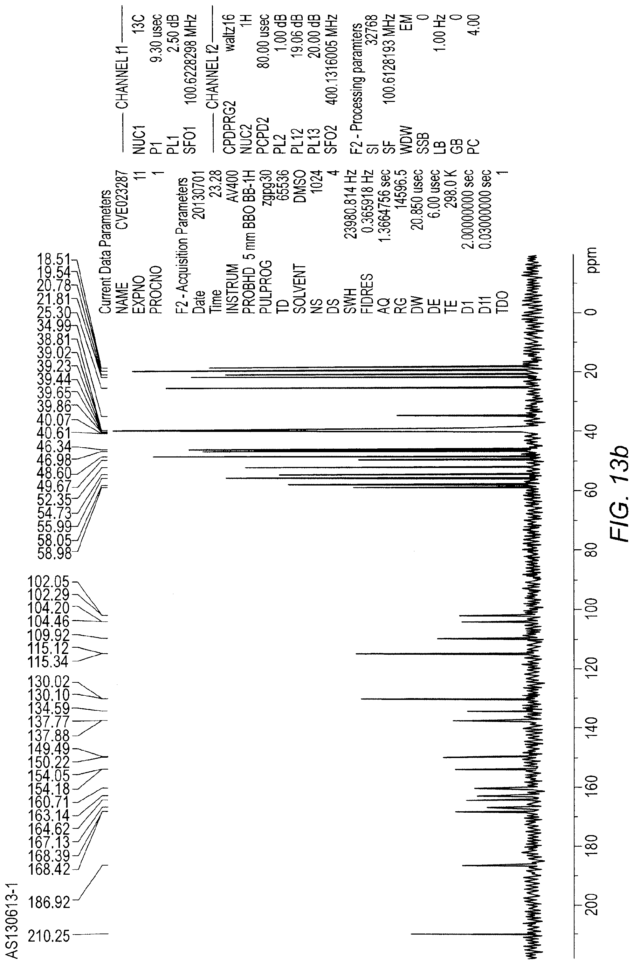

In certain embodiments, the coformer salt is a crystalline Compound (1a) having a ¹³C NMR spectrum corresponding substantially to the spectrum in Figure 13b or a spectrum with peaks corresponding substantially to those in Table A, where entries with 2 peaks represent a doublet:

**Table A**

| **Batch 1** | **Batch 2** | **Batch 3** | **Batch 4** |
|---|---|---|---|
| 21.26 | 21.26 | 21.26 | 21.26 |
| 35.81 | 35.74 | 35.65 | 35.82 |
| 43.15 | 43.13 | 43.11 | 43.15 |
| 59.09 | 59.09 | 59.08 | 59.08 |
| 99.08, 99.32 | 99.05, 99.29 | 99.00, 99.25 | 99.08, 99.33 |
| 103.36, 103.62 | 103.32, 103.59 | 103.28, 103.55 | 103.36, 103.63 |
| 111.67 | 111.68 | 111.70 | 111.66 |
| 115.72, 115.93 | 115.70, 115.91 | 115.66, 115.88 | 115.72, 115.93 |
| 125.94 | 125.95 | 125.95 | 125.94 |
| 128.69 | 128.67 | 128.64 | 128.69 |
| 130.30, 130.42 | 130.31, 130.42 | 130.31, 130.42 | 130.30, 130.41 |
| 130.45, 130.53 | 130.46, 130.55 | 130.48, 130.56 | 130.45, 130.53 |
| 135.35, 135.38 | 135.42, 135.45 | 135.51, 135.54 | 135.34, 135.37 |
| 138.62 | 138.56 | 138.47 | 138.63 |
| 141.03 | 141.10 | 141.20 | 141.02 |
| 145.33 | 145.44 | 145.60 | 145.33 |
| 148.72, 148.85 | 148.73, 148.86 | 148.75, 148.88 | 148.72, 148.84 |
| 149.50 | 149.69 | 149.93 | 149.47 |
| 152.01 | 152.07 | 152.15 | 152.0 |
| 159.36, 159.40 | 159.36, 159.39 | 159.35, 159.39 | 159.36, 159.40 |
| 161.25, 163.69 | 161.24, 163.67 | 161.21, 163.65 | 161.25, 163.69 |
| 164.21, 166.68 | 164.21, 166.68 | 164.20, 166.67 | 164.21, 166.68 |

In certain embodiments, the ¹³C NMR spectrum of crystalline Compound (1a) comprises peaks at 210.3, 25.3, 21.8, 20.8, 19.5, and 18.5 ± 0.2 ppm.

Compound (1a) has a broad endothermal peak on differential scanning calorimetry between 25 °C and about 90 °C and an endotherm with a maximum between about 135 °C and 150 °C, between about 140 °C and 150 °C, or between about 143 °C and 147 °C. Compound (1a) has an endotherm with a maximum between about 135 °C and 150 °C, between about 140 °C and 150 °C, or between about 143 °C and 147 °C.

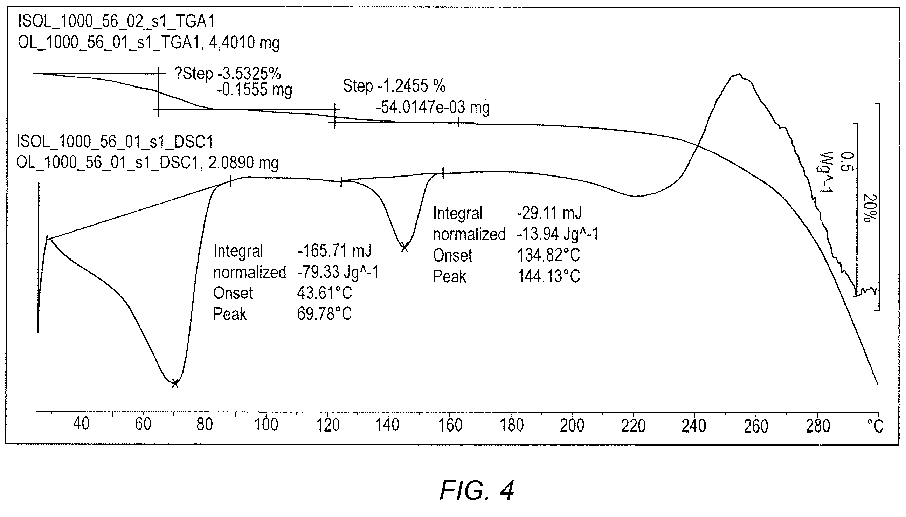

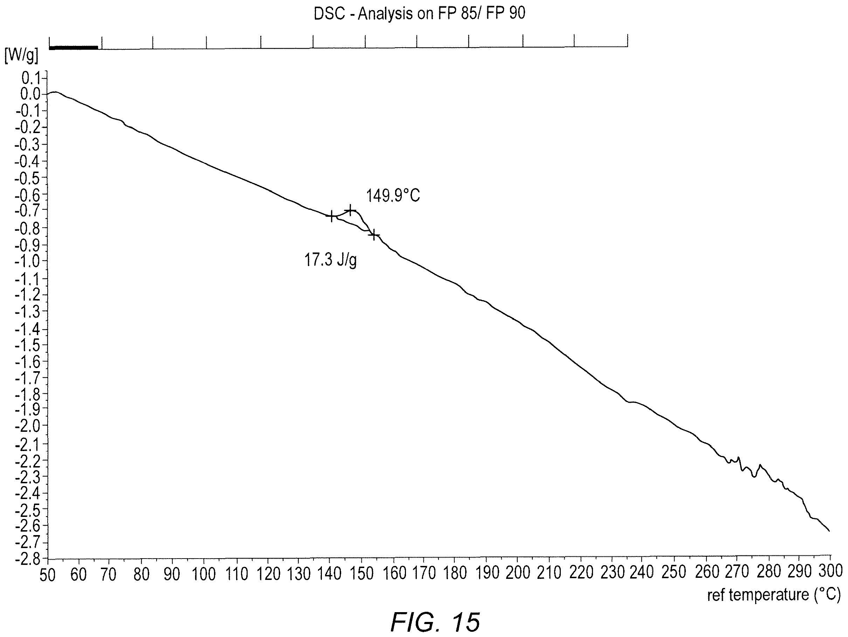

Compound (1a) has a DSC thermogram corresponding substantially to the DSC thermograph of Figures 4 or 15.

Examplified herewith is Compound (1a) having a TGA thermogram indicative of a solvated material. For example, Compound (1a) has a TGA thermogram corresponding substantially to the TGA thermograph of Figure 4. In certain embodiments, crystalline Compound (1a) has a TGA thermogram that exhibits a stepwise weight loss (*e*.*g*., between about 2.5% and 4.5%, between about 3% and 4%, of about 3.5%) when heated from about 25 °C to a temperature of about 90 °C. In certain embodiments, crystalline Compound (1a) has a TGA thermogram that exhibits a gradual mass loss (*e*.*g*., between about 0.5% and 2%, between about 0.75% and 1.75%, between about 1% and 1.5%, of about 1.2%) when heated from about 90 °C to a temperature of about 160 °C.

Compound (1a) according to the present invention has a solid state ¹³C NMR spectrum with peaks at 210.3, 25.3, 21.8, 20.8, 19.5, and 18.5 ppm ± 0.2 ppm. It may additionally be defined by: i. a differential scanning calorimetry thermogram having a broad endotherm between 25 °C and 90 °C and an endotherm with a maximum between about 135 °C and 147 °C; ii. a thermogravimetric analysis thermogram indicative of a solvated material; or iii. a X-ray powder diffraction pattern comprising peaks at 2θ angle degrees ± 0.2 2θ angle degrees of 6.7, 9.7, 18.5, 19.5, and 22.

In another aspect, this disclosure provides a substantially pure (2*S*,3*S*)-methyl 7-fluoro-2-(4-fluorophenyl)-3-(1-methyl-1*H*-1,2,4-triazol-5-yl)-4-oxo-1,2,3,4-tetrahydroquinoline-5-carboxylate (Compound (1)) prepared by treating a coformer salt of Compound (1) with a base and isolating the (2*S*,3*S*)-methyl 7-fluoro-2-(4-fluorophenyl)-3-(1-methyl-1*H*-1,2,4-triazol-5-yl)-4-oxo-1,2,3,4-tetrahydroquinoline-5-carboxylate (Compound (1)). In certain embodiments, the isolated Compound (1) is optionally recrystallized.

### Methods of Preparing Compounds

Provided herein are methods of producing Compound (1) and coformer salts thereof.

In certain embodiments, the methods can provide, for example, improved recoveries of the product, purity of the product, and/or amenability to large scale production, as compared to previously reported syntheses of (2*S*,3*S*)-methyl 7-fluoro-2-(4-fluorophenyl)-3-(1-methyl-1*H*-1,2,4-triazol-5-yl)-4-oxo-1,2,3,4-tetrahydroquinoline-5-carboxylate.

In certain embodiments, the coformer salt of (2*S*,3*S*)-methyl 7-fluoro-2-(4-fluorophenyl)-3-(1-methyl-1*H*-1,2,4-triazol-5-yl)-4-oxo-1,2,3,4-tetrahydroquinoline-5-carboxylate optionally as a solvate and additionally optionally as a hydrate thereof is prepared in a crystalline form resulting in a higher purity of Compound (1) as compared to Compound (1) isolated by chiral chromatography.

In certain embodiments, the preparation of Compound (1) using a coformer is more amenable to large scale production than a preparation using chiral chromatography.

**Scheme A** provides an exemplary outline of the method for making a coformer salt of Compound (1).

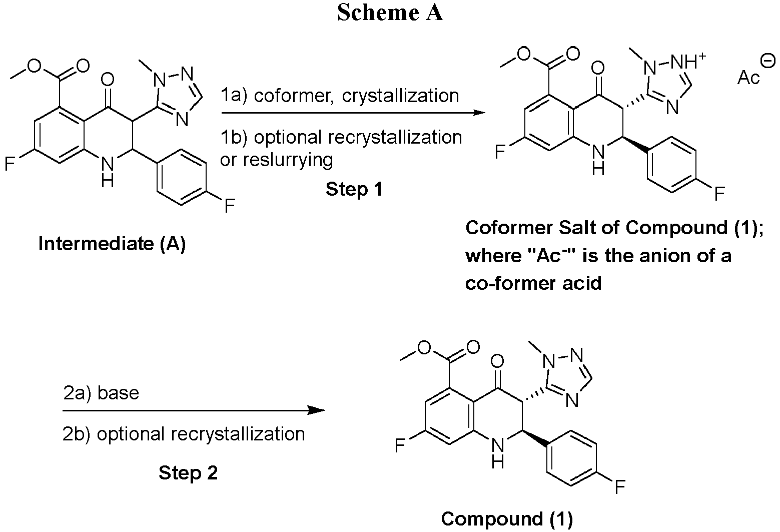

In step 1a), Intermediate (A) can be dissolved at room temperature or at an elevated temperature (a temperature above room temeperature) in one or more step 1a) solvents, where the solvent is sufficient to solubilize Intermediate (A). In certain embodiments, the elevated temperature is at about 30 °C, at about 35 °C, at about 40 °C, at about 45 °C, at about 48 °C, at about 50 °C, at about 52 °C, at about 55 °C, at about 60 °C, at about 65 °C, or at about 70 °C. In certain embodiments, the step 1a) solvent is C₁₋₆ ketone, C₁₋₆ alcohol, ethyl acetate ("EA"), tetrahydrofuran ("THF"), toluene, acetonitrile ("ACN"), heptane, dioxane, or water; or a combination thereof. In certain embodiments, the C₁₋₆ ketone is acetone, methylethylketone ("MEK"), or methylisobutylketone ("MIBK"). In certain embodiments, the C₁₋₆ alcohol is methanol, ethanol, propanol, isopropanol, or butanol. In certain embodiments, the C₁₋₆ alcohol is methanol, ethanol, or isopropanol. In certain embodiments, the step 1a) solvents are ethanol and MIBK; or is the solvents are ethanol, MIBK, and water.

In certain embodiments, the MIBK/ethanol ratio is 5-20/1; or the ratio is 5/1; or 6/1, or 7/1, or 8/1, or 9/1, or 10/1, or 11/1, or 12/1, or 15/1, or 20/1. In certain embodiments, the MIBK/ethanol ratio is 9:1.

In certain embodiments, the MIBK/ethanol/water ratio is 10-15/1-1.5/0.1-0.05; or the ratio is 12-13/1-1.5/0.1-0.05. In certain embodiments, the MIBK/ethanol/water ratio is 13/1.5/0.1; or is 13/1.5/0.05; or is 13/1/0.1; or is 13/1/0.05; or is 12/1.5/0.1; or is 12/1.5/0.05; or is 12/1/0.1; or is 12/1/0.05.

In certain embodiments, in step 1a), Intermediate (A) can be dissolved at an elevated temperature (for example, at about 30 °C, at about 35 °C, at about 40 °C, at about 45 °C, at about 48 °C, at about 50 °C, at about 52 °C, at about 55 °C, at about 60 °C, at about 65 °C, or at about 70 °C), in one or more step 1a) solvent(s) such as acetone, IPA, EA, THF, DMF, toluene, ACN, heptane, dioxane, water, MIBK, MEK, or ethanol, or combinations thereof, to form a step 1a) solution.

In certain embodiments, the step 1a) solvents are MIBK, MEK, water, and/or ethanol. In certain embodiments, the MIBK:MEK:ethanol/water ratio is 20-40:10-20:1-10. In certain embodiments, the MIBK:MEK:ethanol/water ratio is 10-30:20-30:1-5.

In certain embodiments, the step 1a) solvents are MIBK, water, and/or ethanol. In certain embodiments, the step 1a) solvents are MIBK:ethanol:water, with a ratio of 30-50:5-10:1-5, or 35-45:6-7:1-2, or 40:6.5:1.6. In certain embodiments, the MIBK:ethanol:water ratio is 120-130:10-15:0.5-1. In certain embodiments, the step 1a) solvents are MIBK:ethanol, with a ratio of 5-20:1, or 10-20:1, or 20:1, or 19:1, or 18:1, or 10:1, or 9:1, or 8:1.

In certain embodiments, the step 1a) solvents are ethanol and MEK. In certain embodiments, the ratio of ethanol:MEK is 85-99:1-15, or is 90-99:1-10, or is 95-99:1-5, or is 95:5, or is 96:4, or is 97:3, or is 98:2.

In certain embodiments, Intermediate (A) is dissolved in about 5 vol of step 1a) solvent(s), about 7 vol of step 1a) solvent(s), about 10 vol of step 1a) solvent(s), about 12 vol of step 1a) solvent(s), about 14 vol of step 1a) solvent(s), about 16 vol of step 1a) solvent(s), or about 20 vol of step 1a) solvent(s).

The coformer acid (about 1 molar equivalent) can be added and solubilized in the step 1a) solution to produce a step 1a) coformer solution. A solid form of the coformer salt of Compound (1) can be obtained by seeding the step 1a) coformer solution with crystals of the coformer salt of Compound (1), or by cooling the step 1a) coformer solution to about room temperature, about 20 °C, about 15 °C, about 10 °C, about 5 °C, about 0 °C, about - 5 °C, about -10 °C, or about -15 °C. Once the solid coformer salt of Compound (1) has formed, it can be collected by filtration, optionally washed with a step 1a) solvent, and dried.

In step 1b), the coformer salt of Compound (1) can be resuspended in step 1b) solvents to form a step 1b) solution. In certain embodiments, the step 1b) solvents are the same solvent(s) as the step 1a) solvent(s).

In certain embodiments, coformer salt of Compound (1) is resuspended in about 5 vol of step 1a) solvent(s), about 7 vol of step 1a) solvent(s), about 10 vol of step 1a) solvent(s), about 12 vol of step 1a) solvent(s), about 14 vol of step 1a) solvent(s), about 16 vol of step 1a) solvent(s), or about 20 vol of step 1a) solvent(s) at an elevated temperature (for example, at about 30 °C, at about 35 °C, at about 40 °C, at about 45 °C, at about 50 °C, at about 55 °C, at about 60 °C, at about 65 °C, at about 70 °C) to form a step 1b) solution. The step 1b) solution can optionally be cooled to about room temperature, about 20 °C, about 15 °C, about 10 °C, about 5 °C, about 0 °C, about -5 °C, about -10 °C, or about -15 °C to produce a solid form of the coformer salt of Compound (1). The solid coformer salt can be collected by filtration, optionally washed with a step 1b) solvent, and dried.

In step 2a), a base can be added to a solution of the coformer salt of Compound (1) to release Compound (1) and remove the corresponding coformer acid. Any base sufficient to release Compound (1) can be utilized. In certain embodiments, the base is aqueous ammonia (as NH₄OH), NaOH, NaOAc, NaHCOs, or Na₂CO₃. In certain embodiments, the base is aqueous ammonia (as NH₄OH). In certain embodiments, the base is NaOH.

In certain embodiments, the step 2a) solvents can be any solvent or combination of solvents sufficient to solubilize the coformer salt of Compound (1), or that can form a suspension sufficient to allow reaction of the appropriate base to release Compound (1). In certain embodiments, the step 2a) solvents can be any of the step 1a) solvents. In certain embodiments, the step 2a) solvents can be C₁₋₆ ketone, C₁₋₆ alcohol, or water; or a combination thereof. In certain embodiments, the C₁₋₆ ketone is acetone, MIBK, or MEK. In certain embodiments, the C₁₋₆ ketone is acetone. In certain embodiments, the C₁₋₆ alcohol is methanol, ethanol, 2-propanol, or isopropanol. In certain embodiments, the C₁₋₆ alcohol is methanol, 2-propanol, or isopropanol. In certain embodiments, the step 2a) solvents can be acetone, methanol, 2-propanol, isopropanol, or water; or a combiantion thereof. In certain embodiments, the step 2a) solvents can be acetone and methanol; or they can be acetone, methanol, 2-propanol, and water; or they can be acetone, methanol, and isopropanol; or they can be acetone, methanol, isopropanol, and water.

In step 2a), Compound (1) can be released by suspending the coformer salt thereof in step 2a) solvents selected from C₁₋₆ ketone, C₁₋₆ alcohol, and water; or combinations thereof in the presence of a base selected from NH₄OH, NaOH, NaOAc, NaHCOs, or Na₂CO₃; or a combination thereof. In certain embodiments, the step 2a) solvent is acetone, methanol, 2-propanol, isopropanol, or water; or a combiantion thereof, and the base is NH₄OH or aqueous NaOH. In certain embodiments, the base is NH₄OH. In certain embodiments, the step 2a) solvent is acetone, methanol, and isopropanol; and the base is NH₄OH. In certain embodiments, the step 2a) solvent is acetone, methanol, isopropanol, and water; and the base is NH₄OH. In certain embodiments, the step 2a) solvent is acetone, methanol, and 2-propanol; and the base is NH₄OH.

In step 2a), Compound (1) can be released by suspending the coformer salt thereof in about 0.5 to about 10 vol, or about 0.5 to about 5 vol, or about 0.75 to about 2.5 vol of one or more of step 2a) solvent(s) at room temperature or elevated temperature (e.g., about 30 °C, about 32 °C, about 35 °C, about 37 °C, about 38 °C, about 40 °C, about 42 °C, about 45 °C) to form a step 2a) solution and treating the step 2a) solution with about 1 - 1.5 equiv of a suitable base. In some embodiments, the coformer salt is suspended in about 0.75 vol, or about 1 vol, or about 1.5 vol, or about 1.7 vol, or about 2 vol, or about 2.2 vol, or about 2.4 vol, or about 2.5 vol of one or more of step 2a) solvent(s) at room temperature or elevated temperature (e.g., about 30 °C, about 32 °C, about 35 °C, about 37 °C, about 38 °C, about 40 °C, about 42 °C, about 45 °C) to form a step 2a) solution and treating the step 2a) solution with about 1.1 equiv, or about 1.2 equiv, or about 1.3 equiv, or about 1.4 equiv, or about 1.5 equiv of a suitable base. In certain embodiments, the coformer salt is suspended in about 0.5 to about 10 vol, or about 0.5 to about 5 vol, or about 0.75 to about 2.5 vol of one or more the step 2a) solvents selected from acetone, methanol, propanol, isopropanol, and water at room temperature or elevated temperature (*e*.*g*., about 30 °C, about 32 °C, about 35 °C, about 37 °C, about 38 °C, about 40 °C, about 42 °C, about 45 °C) to form a step 2a) solution and treating the step 2a) solution with about 1 - 1.5 equiv of a base selected from NaOH, aqueous NH₃ (optionally, as 25% aqueous NH₃), NaCO₃, NaOAc, and NaHCO₃. In certain embodiments, the coformer salt is suspended in about 0.75 vol, or about 1 vol, or about 1.5 vol, or about 1.7 vol, or about 2 vol, or about 2.2 vol, or about 2.4 vol, or about 2.5 vol of one or more the step 2a) solvents selected from acetone, methanol, propanol, isopropanol, and water of one or more step 2a) solvent(s) at room temperature or elevated temperature (*e*.*g*., about 30 °C, about 32 °C, about 35 °C, about 37 °C, about 38 °C, about 40 °C, about 42 °C, about 45 °C) to form a step 2a) solution and treating the step 2a) solution with about 1 equiv, or about 1.1 equiv, or about 1.2 equiv, or about 1.3 equiv, or about 1.4 equiv, or about 1.5 equiv of a base selected from NaOH, aqueous NH₃ (optionally, as 25% aqueous NH₃), NaCO₃, NaOAc, and NaHCO₃.

In certain embodiments, in step 2a), Compound (1) can be released by suspending the coformer salt thereof in about 0.75 vol, about 1 vol, about 1.5 vol, about 1.7 vol, about 2 vol, about 2.2 vol, or about 2.4 vol of one or more step 2a) solvent(s) such as water, acetone, IPA, and methanol at room temperature or elevated temperature (*e*.*g*., about 30 °C, about 35 °C, about 37 °C, about 38 °C, about 40 °C, about 42 °C, or about 45 °C) to form a step 2a) solution and treating the step 2a) solution with about 1 equiv, about 1.1 equiv, about 1.2 equiv, about 1.3 equiv, or about 1.4 equiv of a base such as NaOH, NH₃ (optionally 25% aqueous NH₃), NaCO₃, NaOAc, or NaHCOs. The pH can optionally be checked and water (0.55 vol) can be added if the pH is ≥ 7. The system can be cooled to about 25 °C, about 30 °C, about 35 °C, or about 40 °C and seed crystals of Compound (1) can optionally be added. Water can be added (3.3 vol) dropwise within about 30 minutes, the suspension cooled within 30 minutes to an internal temperature of about 0 to 5 °C, and the reaction stirred for 15 minutes. The solid form of Compound (1) can be collected by filtration and washed three times with water.

In certain embodiments, the coformer salt is suspended in acetone/isopropanol/methanol in a ratio of about 2-6 vol/1-2 vol/1-2 vol at room temperature or elevated temperature (*e*.*g*., about 30 °C, about 32 °C, about 35 °C, about 37 °C, about 38 °C, about 40 °C, about 42 °C, about 45 °C) to form a step 2a) solution and treating the step 2a) solution with about 1 equiv, or about 1.1 equiv, or about 1.2 equiv, or about 1.3 equiv, or about 1.4 equiv, or about 1.5 equiv of aqueous NH₃ (optionally, as 25% aqueous NH₃). In certain embodiments, the acetone/isopropanol/methanol ratio is about 2-4 vol/1-2 vol/1-2 vol, or is about 2-4 vol/1 vol/1 vol, or is about 2 vol/1 vol/1 vol. In certain embodiments, the coformer salt is suspended in acetone/isopropanol/methanol in a ratio of about 2 vol/1 vol/1 vol at room temperature or elevated temperature (*e*.*g*., about 30 °C, about 32 °C, about 35 °C, about 37 °C, about 38 °C, about 40 °C, about 42 °C, about 45 °C) to form a step 2a) solution and treating the step 2a) solution with about 1.3 equiv aqueous NH₃ (optionally, as 25% aqueous NH₃).

In step 2b), the e.e. of Compound (1) can be improved, if desired, in an optional step by using one or more step 2b) solvent(s) such as water, acetone, IPA, or methanol at about 4 vol, about 5 vol, about 6 vol. or about 7 vol. For example, acetone (4 vol), IPA (1 vol), and methanol (1 vol), can be added to the product of the previous step 2a) and the reaction can be heated to an internal temperature of about 38 °C to 42 °C, about 35 °C, about 38 °C, about 40 °C, about 42 °C, or about 45 °C resulting in a clear step 2b) solution. Water (2 vol) and seed crystals of Compound (1) can be added to the step 2b) solution and the system stirred for about 15 minutes at an internal temperature of about 35 °C. Water can be added dropwise in about 30 minutes. The suspension can then be cooled in 30 min to an internal temperature of about 0 to °5 C and stirred for an additional 15 minutes. The solid can be collected by filtration, washed twice with water, and the chiral purity be determined. The solid can be dried at an internal temperature of about 60 °C under reduced pressure to yield Compound (1).

In certain embodiments, the processes provide substantially pure Compound (1). In certain embodiments, the processes provide Compound (1) with 90-99% e.e., or 95%-99% e.e., or 97%-99% e.e., or ≥ 96%, e.e., or ≥ 97% e.e., or ≥ 98% e.e., or ≥ 99% e.e, or 99.5% e.e.

In another aspect, provided herein is a method of preparing a coformer salt of (2*S*,3*S*)-methyl 7-fluoro-2-(4-fluorophenyl)-3-(1-methyl-1*H*-1,2,4-triazol-5-yl)-4-oxo-1,2,3,4-tetrahydroquinoline-5-carboxylate (Compound (1)), comprising (1) treating methyl 7-fluoro-2-(4-fluorophenyl)-3-(1-methyl-1*H*-1,2,4-triazol-5-yl)-4-oxo-1,2,3,4-tetrahydroquinoline-5-carboxylate with the coformer of the present invention in one or more step 1a) solvent(s) selected from MIBK, MEK, ethanol, and water at an elevated temperature to form a step 1a) solution; (2) allowing the step 1a) solution to stand under conditions sufficient to precipitate the (2*S*,3*S*)-methyl 7-fluoro-2-(4-fluorophenyl)-3-(1-methyl-1*H-*1,2,4-triazol-5-yl)-4-oxo-1,2,3,4-tetrahydroquinoline-5-carboxylate (Compound (1)) as a solid, and in certain embodiments, in a crystalline form; and (3) isolating Compound (1) as a solid, and in certain embodiments, in a crystalline form.

In certain embodiments, the coformer salt is [(1*S*)-*endo*]-(+)-3-bromo-10-camphor sulfonate and the step 1a) solvents are MIBK, water, and ethanol.

In certain embodiments, the method further comprises recrystallizing or reslurrying the coformer salt in one or more step 1b) solvent(s).

The coformer salt of (2*S*,3*S*)-methyl 7-fluoro-2-(4-fluorophenyl)-3-(1-methyl-1*H*-1,2,4-triazol-5-yl)-4-oxo-1,2,3,4-tetrahydroquinoline-5-carboxylate may be in crystalline form after recrystallizing or reslurrying the coformer salt in the one or more step 1b) solvents.

In certain embodiments, the method further comprises suspending the coformer salt of (2*S*,3*S*)-methyl 7-fluoro-2-(4-fluorophenyl)-3-(1-methyl-1*H*-1,2,4-triazol-5-yl)-4-oxo-1,2,3,4-tetrahydroquinoline-5-carboxylate in one or more step 2a) solvent(s) selected from water, acetone, IPA, or methanol at room temperature or elevated temperature to form a step 2a) solution and treating the step 2a) solution with a base selected from NaOH, NH₃ (optionally 25% aqueous NH₃), NaCO₃, NaOAcs, or NaHCO₃; allowing the step 2a) solution to stand under conditions sufficient to precipitate the (2*S*,3*S*)-methyl 7-fluoro-2-(4-fluorophenyl)-3-(1-methyl-1*H*-1,2,4-triazol-5-yl)-4-oxo-1,2,3,4-tetrahydroquinoline-5-carboxylate (Compound (1)) as a solid, and in certain embodiments, in a crystalline form; and (3) isolating Compound (1) as a solid, and in certain embodiments, in a crystalline form.

In certain embodiments, the method further comprises recrystallizing or reslurrying Compound (1) in one or more step 2b) solvent(s). In certain embodiments, Compound (1) is in crystalline form after recrystallizing or reslurrying the coformer salt in the one or more step 2b) solvents.

### PREPARATION OF COMPOUNDS

In certain embodiments, the method of preparing a coformer salt of Compound (1) comprises any of the various embodiments described above and below.

The compounds disclosed herein are commercially available or can be readily prepared from commercially available starting materials according to established methodology in the art of organic synthesis. General methods of synthesizing the compounds of this disclosure can be found in, *e*.*g*., Stuart Warren and Paul Wyatt, Workbook for Organic Synthesis: The Disconnection Approach, second Edition, Wiley, 2010. Synthesis of some of the compounds are exemplified in detail below.

In certain embodiments, individual stereoisomers of the compounds of this disclosure are prepared synthetically from commercially available starting materials that contain asymmetric or chiral centers or by preparing racemic mixtures that are subsequently stereoselectively separated into enantiomers. Stereoselective separation methods include, for example, (1) attachment of an enantiomer mixture to a chiral auxiliary, separation of the resulting mixture of diastereomers by recrystallization or chromatography and liberation of an optically pure product from the auxiliary or (2) direct separation of the mixture of optical enantiomers on a chiral chromatographic column.

### X-Ray Powder Diffraction (XRPD)

Unless otherwise specified, when an XRPD peak is expressed in 2θ angle degrees, it should be understood that copper Kα1 radiation was used.

In certain embodiments, the 2θ angle degrees value provided herein varied to an extent of about ± 0.2 °θ, while still describing the same XRPD peak.

XRPD Procedure 1: X-Ray Powder Diffraction patterns were collected on a Bruker AXS C2 GADDS diffractometer using Cu Kα radiation (40 kV, 40 mA), automated XYZ stage, laser video microscope for auto-sample positioning and a HiStar 2-dimensional area detector. X-ray optics consisted of a single Göbel multiplayer mirror coupled with a pinhole collimator of 0.3 mm. A weekly performance check was carried out using a certified standard NIST 1976 Corundum (flat plate). The beam divergence, *i*.*e*., the effective size of the X-ray beam on the sample, was approximately 4 mm. A Θ-Θ continuous scan mode was be employed with a sample-detector distance of 20 cm which gives an effective 2Θ range of 3.2 ° to 29.7 °. Typically samples were exposed to the X-ray beam for 120 seconds. GADDS for XP/2000 4.1.43 software was used for data collection and Diffrac Plus EVA v13.0.0.2 or v15.0.0.0 software was used for data analysis and presentation. Ambient conditions: Samples run under ambient conditions were prepared as flat plate specimens using powder as received without grinding; approximately 1-2 mg of the sample were lightly pressed on a glass slide to obtain a flat surface. Non-ambient conditions: Samples run under non-ambient conditions were mounted on a silicon wafer with heat-conducting compound. The samples were then heated to the appropriate temperature at 10 °C/min and subsequently held isothermally for 1 minute before initiation of data collection.

XRPD Procedure 2: Alternatively, X-Ray Powder Diffraction patterns were collected on a Bruker D8 diffractometer using Cu Kα radiation (40 kV, 40 mA), Θ-2Θ goniometer, and divergence of V4 and receiving slits, a Ge monochromator and a Lynxeye detector. The instrument was performance-checked using a certified Corundum standard (NIST 1976). Diffrac *Plus* XRD Commander v.2.6.1 software was used for data collection and Diffrac *Plus* EVA v13.0.0.2 or v15.0.0.0 software was used for data analysis and presentation. Samples were run under ambient conditions as flat plate specimens using powder as received. The sample was gently packed into a cavity cut into polished, zer0background (510) silicon wafer. The sample was rotated in its own plane during analysis. Data collection details included: angular range of 2 to 42 °2Θ, step size of 0.05 °2Θ, and collection time of 0.5 s/step.

### Single Crystal X-ray Diffraction (SCXRD)

Data was collected on an Oxford Diffraction Supernova Dual Source, Cu at Zero, Atlas CCD diffractometer equipped with an Oxford Cryosystems Cobra cooling device. The data was collected using MoKα radiation. Structures were typically solved using either the SHELXS or SHELXD programs and refined with the SHELXL program, which is a part of the Bruker AXS SHELXTL suite (V6.10). Hydrogen atoms attached to carbon can were placed geometrically and were typically allowed to refine with a riding isotropic displacement parameter. Hydrogen atoms attached to a heteroatom were located in a difference Fourier synthesis and were typically allowed to refine freely with an isotropic displacement parameter.

### Nuclear Magnetic Resonance

For examples 1-3 and 5, NMR spectra were collected on a Bruker 400 MHz instrument equipped with an auto-sampler and controlled by a DRX400 console. Automated experiments can be acquired using ICON-NMR v4.0.7 running with Topspin v1.3 using the standard Bruker loaded experiments. For non-routine spectroscopy, data was acquired through the used of Topspin alone. Data was reported as follows in ppm (δ): chemical shift (multiplicity, integration, coupling constant in Hz).

In the ¹³C solid state NMR, the peak positions can vary depending on factors such as signal-to-noise ratio, peak width, temperature, spinning speed, decoupling efficiency, magic angle setting, data processing procedures and parameters, and software peak picking algorithm. In addition, peak position is relative to the chemical shift referencing procedure. Several different chemical shift reference standards can be used and will not necessarily give the same results. Use of different chemical shift reference standards can lead to peak positions that are separated by several ppm. However, typically all of the peaks will have a systematic change in position in the same direction if a different reference standard is used or if the analyst uses a different value for the reference peak position of the same standard.

In certain embodiments, the ppm values in the ¹³C solid state NMR provided herein varied to an extent of about ± 0.2 ppm, while still describing the same peak.

### Differential Scanning Calorimetry (DSC)

DSC data was collected on a Mettler DSC 823E equipped with a 34 position auto-sampler. The instrument was calibrated for energy and temperature using certified indium. Typically 0.5-2 mg of each sample, in a pin-holed aluminum plan, was heated at 10 °C/min from 25 °C to 300 °C. A nitrogen purge at 50 mL/min was typically maintained over the sample. STARe v9.20 software was used as the instrument control and data analysis software.

### Thermo-gravimetric Analysis (TGA)

TGA data was collected on a Mettler TGA/SDTA 851e equipped with a 34 position auto-sampler. The instrument was temperature calibrated using certified indium. Typically, 3-6 mg of each sample was loaded onto a pre-weighed aluminum crucible and heated at 10 °C/min from ambient temperature to 350 °C. A nitrogen purge at 50 mL/min was maintained over the sample.

### IR Spectrum

IR data was collected on a Perkin Elmer Spectrum One FT-IR Spectrometer with a Universal ATR Sampling Accessory and a pyroelectric DTGS detector (deuterated Triglycine sulfate).

### Chiral Purity Determination by HPLC

Chiral HPLC analysis was performed on an Agilent HP1100 series system equipped with a diode array detector and using ChemStation software vB.02.01-SR1 or SR2 using the methods detailed below:

**Chiral HPLC Method Parameters for Analysis of Methyl 7-fluoro-2-(4-fluorophenyl)-3-(1-methyl-1H-1,2,4-triazol-5-yl)-4-oxo-1,2,3,4-tetrahydroquinoline-5-carboxylate**

| | |
|---|---|
| Sample Preparation | 1.0 mg/mL in DCM |
| Column | Chiralpak IC, 250 x 4.6 mm |
| Column Temperature (°C) | 35 |
| injection (L) | 10 |
| Detection: Wavelength bandwidth (nm) | 235,4 |
| Flow rate (mL/min) | 1.0 |
| Phase A | 20%/80% EtOH/Hexane |
| Phase B | N/A |

### SYNTHETIC EXAMPLES

### Example 1: Salt Screen on Intermediate (A)

Coformers in Table 1, which were supplied or prepared as salts, were eluted on ion exchange resins in order to isolate their free acid counterpart. However, coformers containing sulfuric acid were not used directly as free acids due to the free acids' chemical instability. Instead, coformers containing sulfuric acid were dissolved as salts in an appropriate solvent and one molar equivalent of HCl for each sulfuric acid group was added (4 N HCl in dioxane). Coformer compounds Ac4, Ac18, Ac20, Ac38, Ac69, Ac70, Ac75, Ac110-Ac127 of Table 1 serve as reference compounds.

Coformers Ac20, Ac125 and Ac69 were added as free acid solids
Coformers Ac38, Ac49, Ac111, Ac18, and Ac115 were added as free acids in a solution of ethanol at a concentration of 5 M, 1 M, 1 M, 5 M, and 5 M, respectively. The following coformers were added as free acids in solutions in aqueous ethanol: Ac70 (10% v/v, 0.45 M), Ac75 (10% v/v, 0.45 M), Ac126 (25% v/v, 0.8 M), Ac4 (monohydrate, 7% v/v, 1 M), Ac117 (20% v/v, 0.4 M), Ac116 (10% v/v, 0.45 M), and Ac127 (35% v/v, 0.5 M). The following coformers were added as sodium salts in solutions (in addition to the one molar equivalent of 4 N HCl in dioxane): Ac118 (0.8 M in ethanol), Ac110 (5 M in ethanol), Ac113 (3.7 M in THF), Ac114 (0.8 M in 80% by volume aqueous THF), and Ac119 (1.3 M in 25% by volume aqueous THF). Coformer Ac120 was added as a free acid in a 0.5 M solution of water. The following coformers were added as ammonium salts in solutions (in addition to the molar equivalent of 4 N HCl in dioxane): Ac121 (bis-ammonium salt, 0.7 M in 38% by volume aqueous THF), Ac122 (1.4 M in water), Ac112 (0.5 M in water), Ac123 (1 M in 50% aq. THF), and Ac124 (1.3 M in water).

**Table 1. Coformers**

| **Acid ID** | **Resolving Agent** | **Structure** |
|---|---|---|
| Ac20 | *R*-(-)-1,1'-binaphthyl-2,2'-diyl hydrogenphosphate | 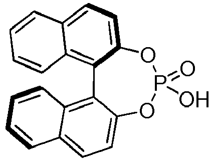 |
| Ac38 | *R*-(+)-alpha-methoxy-alpha-(trifluoromethyl) phenyl acetic acid | 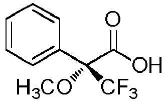 |
| Ac49 | [(1*S*)*-endo*]-(+)-3-bromo-10-camphor sulfonic acid monohydrate | 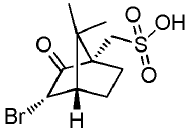 |
| Ac70 | *S*-chlorophos (CAS Reg. No. 98674-86-3) | 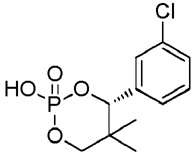 |
| Ac75 | *R-*2-methoxy cyclophos | 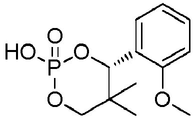 |
| Ac111 | 2'-hydroxyspiro[bicyclo[2.2.1]hept[5]ene-2,5'-[1,3,2]dioxaphosphinane] 2'-oxide | 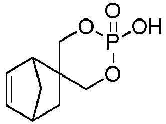 |
| Ac115 | (1*S*,5*R*)-5-(2-acetamidopropan-2-yl)-2-methylcyclohex-2-ene-1-sulfonic acid | 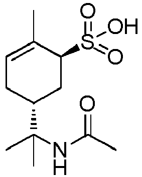 |
| Ac117 | 2-acetamido-2-((1*S*)-4-methyl-5-oxocyclohex-3-en-1-yl)propane-1-sulfonic acid | 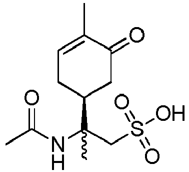 |
| Ac118 | sodium [(1*R*,3*E*)-3-benzylidene-7,7-dimethyl-2-oxobicyclo[2.2.1]heptan-1-yl]methanesulfonate | 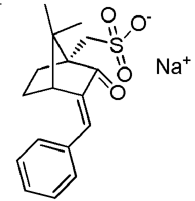 |
| Ac 120 | (*R*)-carboxy(phenyl)methyl sulfate | 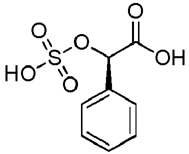 |
| Ac121 | deoxycholic acid diammonium 3,12 dislfate | 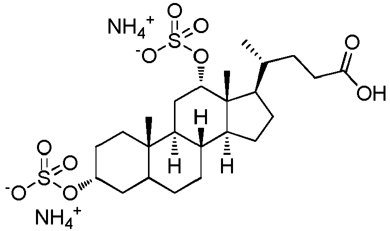 |
| Ac 122 | (1*R*,2*S*,5*R*)-5-methyl-2-(prop-2-yl)cyclohexyl sulfate | 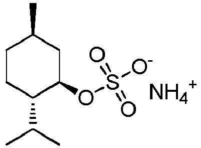 |
| Ac112 | lithocholic acid ammonium 3-sulfate | 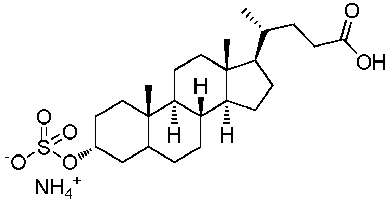 |
| Ac110 | (1*S*)-phenylethanesulfonic acid | 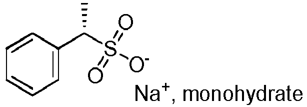 |
| Ac116 | {(4*S*)-4-[2-(acetylamino)propan-2-yl]cyclohex-1-en-1-yl}methanesulfonic acid | 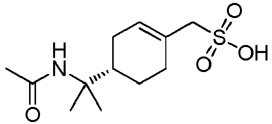 |
| Ac113 | sodium [(4*S*)-4-(propan-2-yl)cyclohex-1-en-1-yl)methane sulfonate | 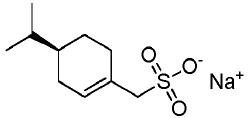 |
| Ac114 | sodium (1*S*,5*R*)-2-methyl-5-(propan-2-yl)cyclohex-2-ene-1-sulfonate | 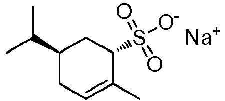 |
| Ac119 | sodium [(1*R*,3*E*)-3-(4-methoxybenzylidene)-7,7-dimethyl-2-oxobicyclo[2.2.1]hept-1-yl)methanesulfonate | 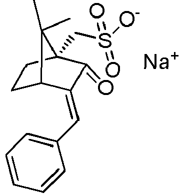 |
| Ac123 | cholesterol ammonium 3-sulfate | 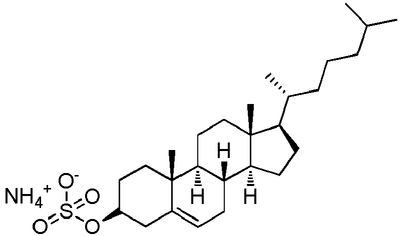 |
| Ac124 | ammonium (2*S*)-1,7,7-trimethylbicyclo[2.2.1]hept-2-yl sulfate | 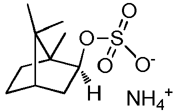 |
| Ac125 | [(2*E*,3*S*)-3-bromo-1,7-dimethyl-2-[2-(phenylsulfonyl)hydrazinylidene]bicycl o[2.2.1]hept-7-yl]methanesulfonic acid | 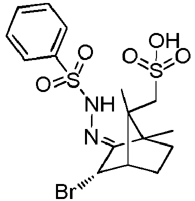 |
| Ac127 | [(2Z)-7,7-dimethyl-2-[2-(phenylsulfonyl)hydrazinylidene]bicycl o[2.2.1]hept-7-yl]methanesulfonic acid | 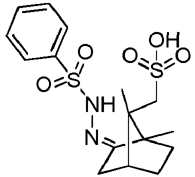 |
| Ac126 | (1*S*)-(endo, anti)-(-)-3 -bromo-camphor-8-sulfonic acid | 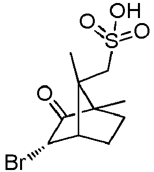 |
| Ac4 | diisopropylidene-2-keto-L-gulonic acid ((-)-2,3,4,6-di-O-isopropylidene-2-keto-L-gulonic acid monohydrate) | 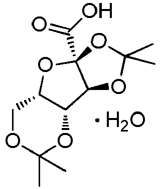 |
| Ac18 | (1*S*)-camphor-10-sulphonic acid | 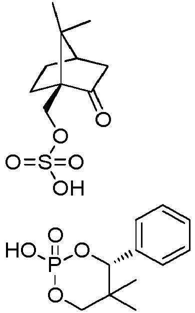 |
| Ac69 | *R*-chlorophos | 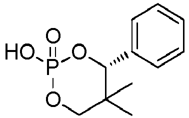 |

Clear solutions of Intermediate (A) (30 or 50 mg) at 50 °C in ethanol (20 vol.), MEK (40 vol.), and MIBK (20 vol.) were prepared. The coformer acids (1.2 mol equiv), prepared as described in the preceding paragraph, were added at 50 °C and slurried for about 1-2 hour. The suspensions were cooled to room temperature and slurried at room temperature for 2 days. Clear solutions were successively cooled to 5 °C, 20 °C and submitted to slow evaporation. Gums were submitted to maturation cycles (temperature cycling).

**Table 2. Attempted Conditions to Obtain Crystalline Coformer Salts of Compound (1): (2S,3S)-methyl 7-fluoro-2-(4-fluorophenyl)-3-(1-methyl-1H-1,2,4-triazol-5-yl)-4-oxo-1,2,3,4-tetrahydroquinoline-5-carboxylate**

| **Acid ID** | **Solvent for Intermed. A** | **Solid after Cooling to 4 or 5 °C?** | **Solid after Cooling to** - **20 °C,** 2 **days?** | **Solid after Evap.?** | **Cmpd (1) by HPLC on Liquid Phase** | **Cmpd (1) by HPLC on Solid Phase** |
|---|---|---|---|---|---|---|
| Ac20 | EtOH | Suspension | - | - | 52% | - |
| | MEK | Suspension | - | - | 52% | - |
| | MIBK | Suspension | - | - | 50% | - |
| Ac38 | EtOH | Suspension | - | - | 55% | 50% |
| | MEK | Clear solution | Clear solution | - | - | - |
| | MIBK | Clear solution | Light suspension | - | 50% | - |
| Ac49 | EtOH | Clear solution | Light suspension | - | 32% | 84% |
| | MEK | Clear solution | Clear solution | - | - | - |
| | MIBK | Suspension | - | - | 23% | 95% |
| Ac70 | EtOH | Suspension | - | - | 59% | 49% |
| | MEK | Clear solution | Clear solution | Yes | 45% | 49% |
| | MIBK | Clear solution | Clear solution | Yes | 49% | - |
| Ac75 | EtOH | Suspension | - | - | 51% | - |
| | MEK | Clear solution | Clear solution | Yes | 46% | 48% |
| | MIBK | Clear solution | Clear solution | Yes | 49% | - |
| Ac111 | EtOH | Suspension | - | - | 50% | - |
| | MEK | Clear solution | Clear solution | - | - | - |
| | MIBK | Clear solution | Clear solution | Yes | 50% | - |
| Ac115 | EtOH | Light suspension | - | - | 48% | - |
| | MEK | Clear solution | Clear solution | - | - | - |
| | MIBK | Gum | - | - | - | - |
| Ac117 | EtOH | Clear solution | Clear solution | Yes | 50% | - |
| | MEK | Suspension | - | - | 51% | - |
| | MIBK | Suspension | - | - | 52% | - |
| Ac 120 | EtOH | Light suspension | - | - | 51% | - |
| | MEK | Clear solution | Clear solution | Yes | 46% | 51%- |
| | MIBK | Clear solution | Suspension | - | 49% | - |
| Ac116 | EtOH | Clear solution | Clear solution | Yes | 46% | 50% |
| | MEK | Suspension | - | - | 51% | - |
| | MIBK | Suspension | - | - | 50% | - |
| Ac110 | EtOH | Clear solution | Clear solution | Yes | - | - |
| | MEK | Clear solution | Clear solution | Yes | 32% | 98% |
| | MIBK | Suspension | - | - | 17% | 96% |
| Ac118 | EtOH | Clear solution | Clear solution | - | - | - |
| | MEK | Clear solution | Clear solution | - | - | - |
| | MIBK | Clear solution | Clear solution | - | - | - |
| Ac121 | EtOH | Clear solution | Clear solution | Yes | 48% | - |
| | MEK | Light suspension | - | - | 50% | - |
| | MIBK | Gum | - | - | - | - |
| Ac122 | EtOH | Suspension | - | - | 51% | - |
| | MEK | Suspension | - | - | 50% | - |
| | MIBK | Suspension | - | - | 52% | - |
| Ac122 | EtOH/H₂O/ dioxane | Yes | - | - | 51-52% | - |
| Ac112 | EtOH | Clear solution | Light suspension | - | 50% | - |
| | MEK | Light suspension | - | - | 52% | - |
| | MIBK | Suspension | - | - | 51% | - |
| Ac113 | EtOH | - | - | Yes | 50% | - |
| | MEK | - | - | - | - | - |
| | MIBK | - | - | - | - | - |
| Ac114 | EtOH | - | - | Yes | 54% | 39% |
| | MEK | - | - | Yes | 50% | - |
| | MIBK | - | Yes | - | 48% | - |
| Ac119 | EtOH | - | - | Yes | 50% | - |
| | MEK | - | - | - | - | - |
| | MIBK | - | - | - | - | - |
| Ac123 | EtOH/THF/ H₂O/ dioxane | - | Suspension | - | 49% | - |
| Ac124 | EtOH/H₂O/ dioxane | Suspension | Suspension | - | 49-50% | - |
| Ac125 | EtOH | Yes | - | - | 49% | - |
| | MEK | Yes | - | - | 50% | - |
| | MIBK | Yes | - | - | 50% | - |
| Ac127 | EtOH | - | - | - | - | - |
| | MEK | - | - | - | - | - |
| | MIBK | - | - | Yes | 53% | 49% |
| Ac126 | EtOH | - | - | Yes | 50% | - |
| | MEK | - | - | - | - | - |
| | MIBK | - | - | - | - | - |
| Ac4 | EtOH | - | - | Yes | 48% | - |
| | MEK | - | - | Yes | 50% | - |
| | MIBK | - | - | Yes | 50% | - |
| Ac18 | EtOH | Yes | - | - | 51% | - |
| | MEK | - | - | Yes | 51% | - |
| | MIBK | Yes | - | - | 51% | - |
| Ac69 | EtOH | Yes | - | - | 49% | - |
| | MEK | Yes | - | - | 50% | - |
| | MIBK | Yes | - | - | 50% | - |

Scheme 1 below describes use of Ac49 as a coformer acid for the preparation of Compound (1a) and for the chiral resolution of Compound (1).

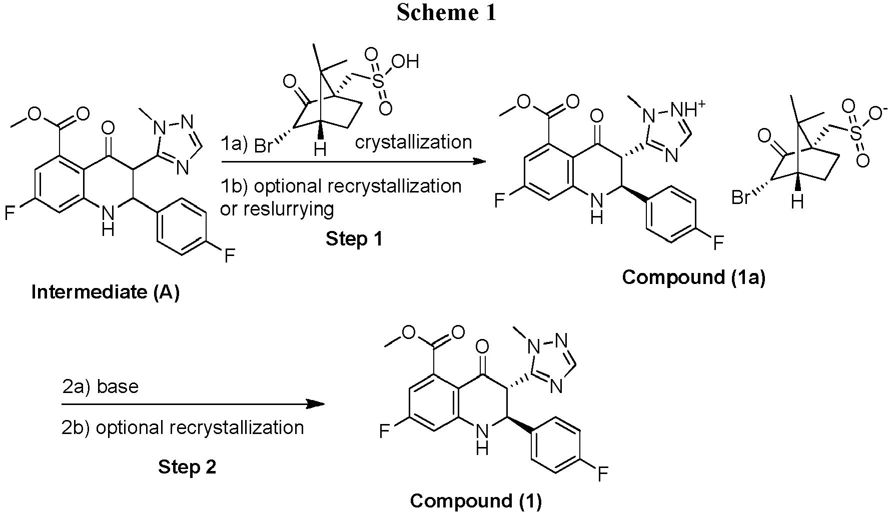

### Example 2 - Preparation of Compound (1) Using Scheme 1

### Step 1a

Intermediate (A) (5 g, 12.5 mmol) was dissolved in 9:1 v/v MIBK/ethanol (70 mL, 14 vol.) at 50 °C with stirring and dissolution was observed in less than about 5 minutes. [(1*S*)-*endo*]-(+)-3-bromo-10-camphor sulfonic acid monohydrate (4.1 g, 12.5 mmol) was added and dissolution was observed in about 10-20 minutes. Seeding was then performed with Compound (1a) (95% e.e., 5 mg, 0.1% w.) and the system was allowed to equilibrate for about 1 hour at 50 °C, was cooled to about 20 °C at 0.15 °C/min, and then equilibrated at 20 °C for 2 hours. The solid phase was isolated by filtration, washed with ethanol, and dried at about 50 °C and 3 mbar for about 2 to 3 hours to yield Compound (1a) as a 0.6 molar equiv. EtOH solvate and 0.6 molar equiv. hydrate (93.4% e.e.).

### Step 1b

Compound (1a) was then suspended in MIBK/ethanol 95/5% by volume (38 mL, 10 vol.) at 50 °C with stirring. After about 2 hours at 50 °C, the suspension was cooled to about 5 °C for 10 to 15 hours. The solid phase was recovered by filtration and dried at about 50 °C and 3 mbar for about 3 hours. Compound (1a) (97.4% e.e.) was recovered.

### Step 2

Compound (1) was released by suspending Compound (1a) (3.9 g, 5.5 mmol), without performing the optional reslurrying in Step 1, in 20 mL of water at room temperature and treating with 5M sodium hydroxide in water (1.3 mL, 1.2 mol). The mixture was kept at room temperature for about 15 hours and the solid was isolated by filtration and dried at 50 °C and 3 mbar for about 3 hours. Compound (1) was recovered (94.4% e.e.).

### Example 3 - Large Scale Preparation of Compound (1) Using Scheme 1

The procedure of Example 1 was followed using 3.3 kg of Intermediate (A) and the respective solvent ratios to provide 95.7% e.e. in Step 1a; 99.2% e.e. in Step 1b; and 99.2% e.e. in Step 2.

### Example 4 - Alternative Preparation of Compound (1) Using Scheme 1

### Step 1a

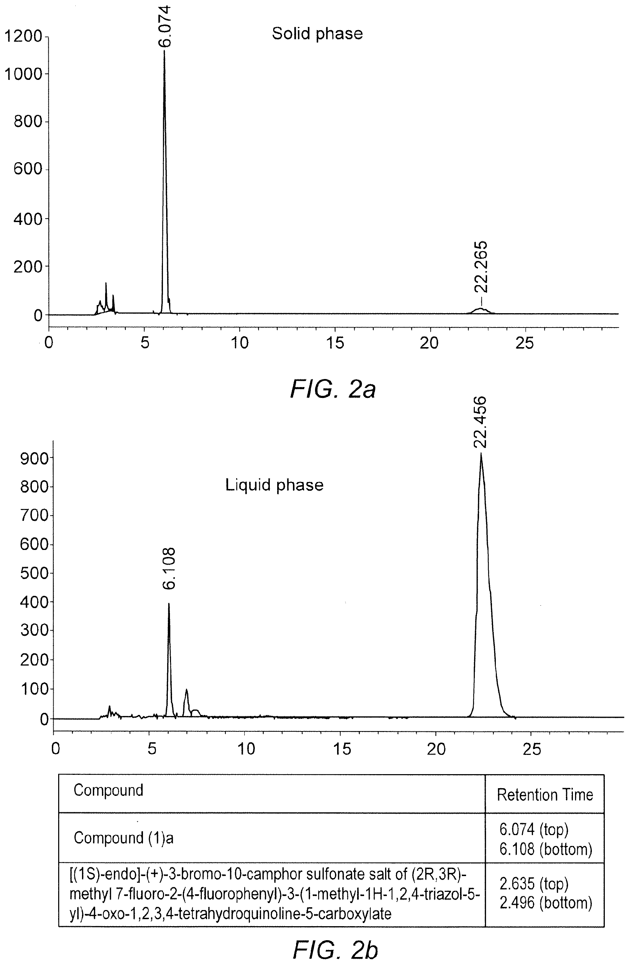

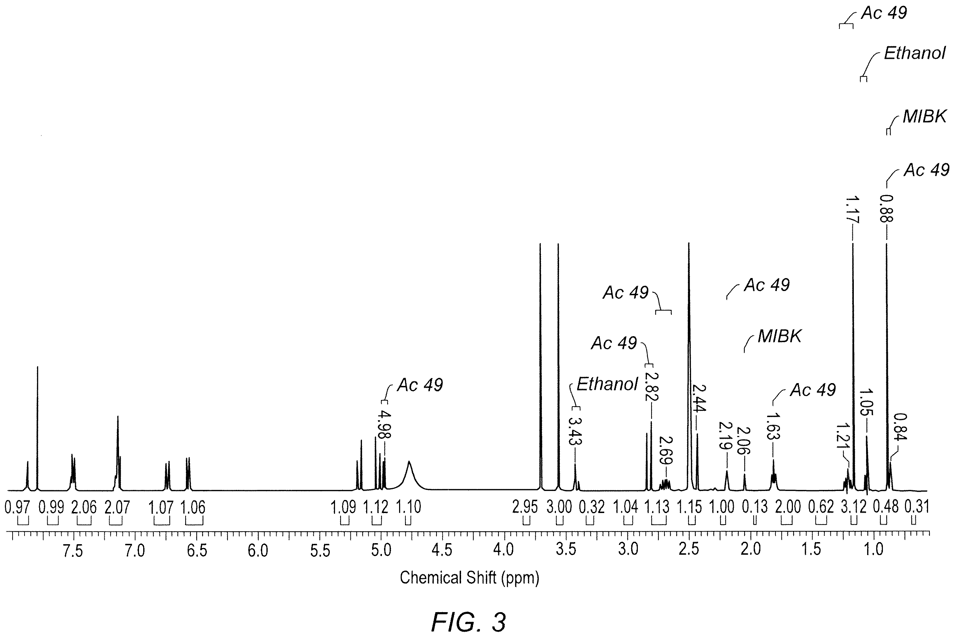

Intermediate (A) (751 mg, 1.86 mmol)) was dissolved in 9:1 v/v MIBK/ethanol (7.5 mL, 10 vol.) at 50 °C with stirring. [(1*S*)-*endo*]-(+)-3-bromo-10-camphor sulfonic acid monohydrate (620 mg, 1.88 mmol, 1 equiv.) was added. Formation of a precipitate was observed at about 1 hour at 50 °C. The system was then cooled to about 5 °C at 0.1 °C/min, and then equilibrated at 5 °C for about 60 hours. The solid phase was isolated by filtration and dried at about 50 °C and 3 mbar for about 2 hours to yield Compound (1a)(92% e.e.). *See* Figures 1-4 for XRPD (Figure 1), chiral HPLC (Figure 2), ¹H NMR (Figure 3), and TGA/DSC analyses (Figure 4). The XRPD pattern from the material in Example 3 is similar to that in Example 1 with some slight shifts in the positions of specific diffraction peaks (highlighted by black arrows in Figure 1). The ¹H NMR was consistent with a mono-salt of Compound (1a) containing 0.5 molar equivalent of EtOH and 0.6% by weight residual MIBK. The TGA analysis showed a stepwise mass loss of 3.5% between 25 and 90 °C (potentially representing loss of the 0.5 molar equivalent of EtOH) and a gradual mass loss of 1.2% between 90 and 160 °C (potentially representing the loss of adsorbed water). The DSC analysis had a broad endotherm between 25 and 90 °C representing desolvation and an endotherm at 135 °C representing melt/degradation.

### Step 1b

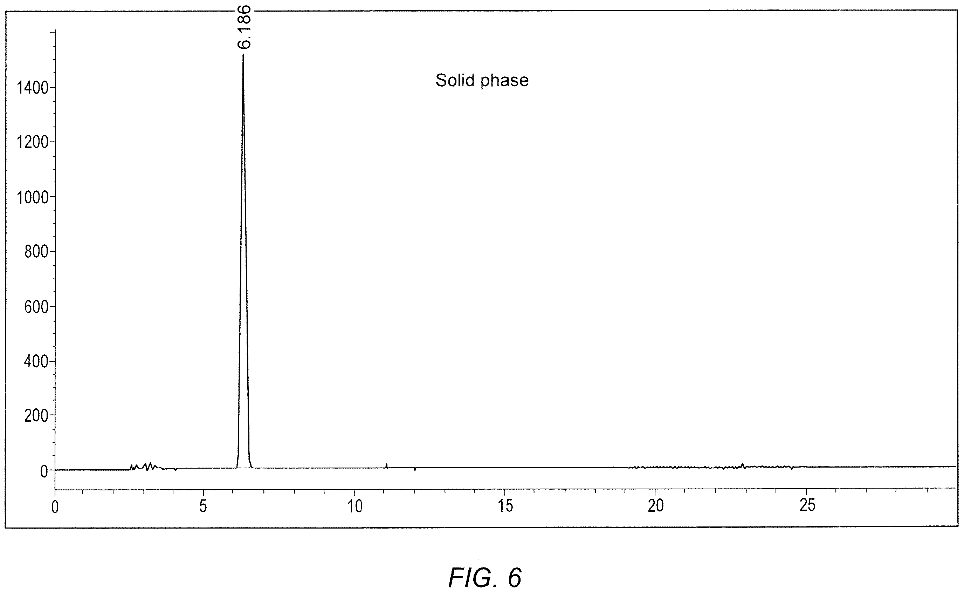

Compound (1a) (100.3 mg, 0.141 mmol) was re-suspended in 95:5 v/v MIBK/EtOH (1 mL, 10 vol.) at 50 °C and stirred for 1 hour before cooling to 5 °C at 0.1 °C/min. The solid (99.4% e.e.) was recovered by filtration after 1 night at 5 °C. Shifts in the XRPD diffraction peaks were no longer detected (Figure 5; compare Figure 1). Figure 6 shows the chiral HPLC for Compound (1a).

### Step 2

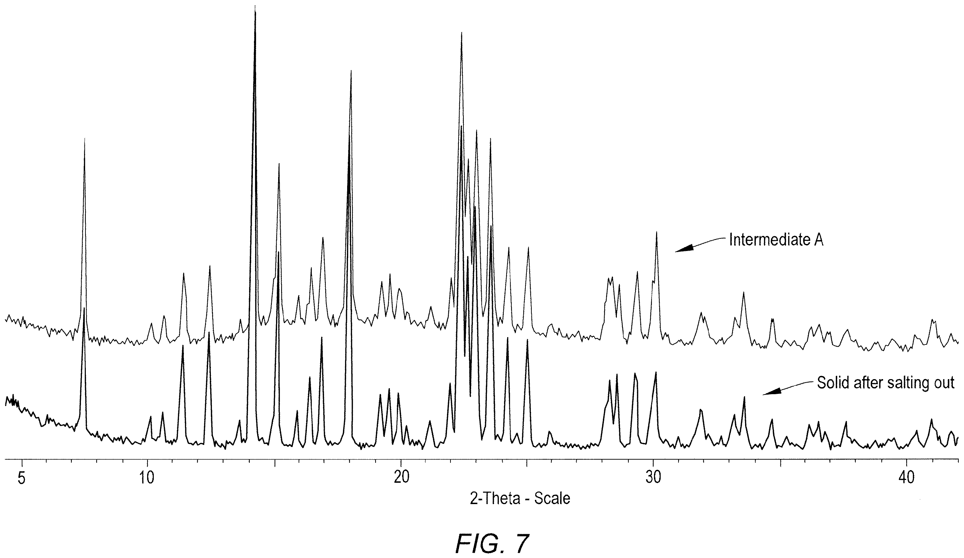

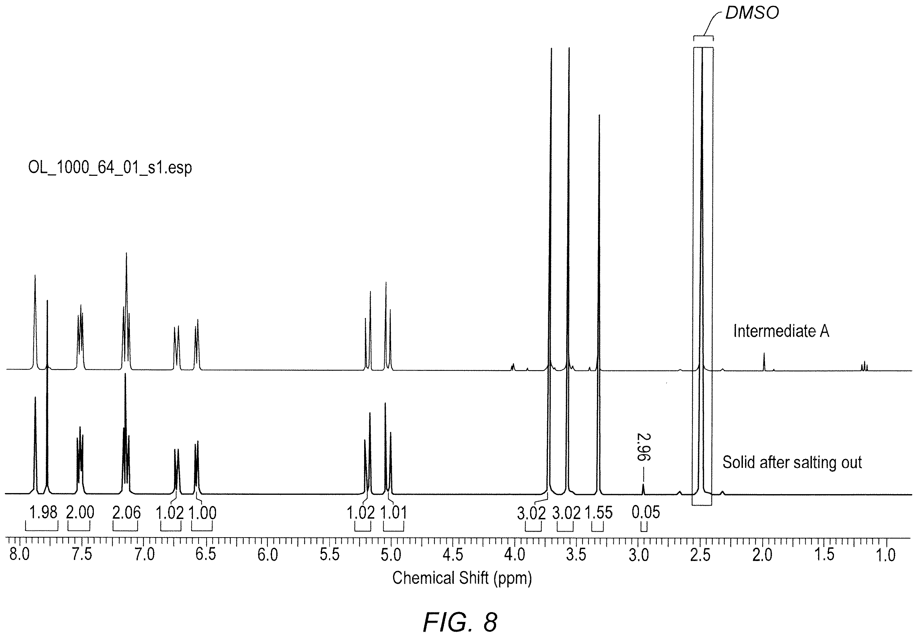

Compound (1a) (100.2 mg, 0.141 mmol) from Step 1a was suspended in water (2 mL, 20 vol.) at 50 °C and 5 M NaOH in water (34 µL, 1.2 molar equiv) was added. The resulting suspension was kept at 50 °C for one night, cooled to room temperature (uncontrolled cooling) and filtered to yield Compound (1) (92% e.e.). The chiral purity was not impacted by this step and no [(1*S*)-*endo*]-(+)-3-bromo-10-camphor sulfonic acid was detected by NMR. Figure 7 compares the XRPD of Compound (1) in Step 2 with Intermediate (A), the starting material of Step 1. Figure 8 shows the NMR of Compound (1) in Step 2 with Intermediate (A), the starting material of Step 1.

### Example 5 - Alternative Preparation of Compound (1) Using Scheme 1 Step 1a

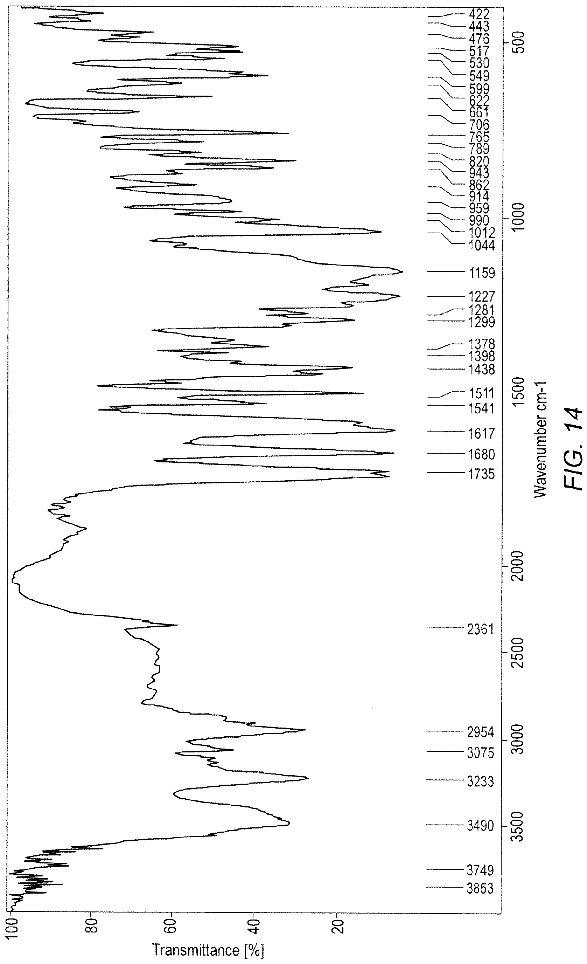

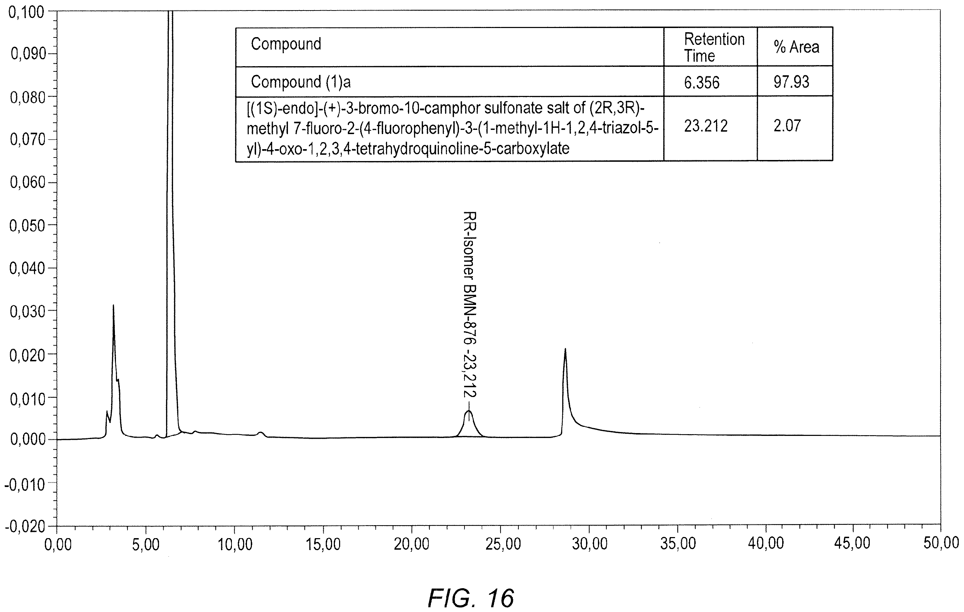

Intermediate (A) (1 equiv.) was added with stirring to a solution of MIBK (12-13 vol), ethanol (1-1.5 vol), and water (0.05-0.10 vol) and the reaction was heated within 15 minutes to an internal temperature of about 48 °C to about 52 °C . [(1*S*)-*endo*]-(+)-3-bromo-10-camphor sulfonic acid (1 equiv) was added and the reaction was stirred for about 5-10 mins at an internal temperature of about 48 °C to about 52 °C until dissolution occurred. Seed crystals of Compound (1a) were added and the reaction was allowed to proceed for 1 hour at an internal temperature of about 48 °C to about 52 °C. The reaction was cooled at a rate of 0.15 °C /min to about 19-21 °C. The suspension was stirred for 2 hours at an internal temperature of about 19 °C to 21 °C and then was collected by filtration and washed twice with ethanol. The product was characterized by ¹H NMR and ¹³C NMR (Figures 13a and 13b), IR Spectrum (Figure 14), DSC (Figure 15), and chiral HPLC (Figure 16).

### Step 2a

To Compound (1a) (1 equiv.) was added acetone (1.1 vol), IPA (0.55 vol), and methanol (0.55 vol) and the reaction was heated to an internal temperature of about 38 °C to 42 °C. Aqueous ammonia (25%) (1.3 equiv) was added and the reaction was stirred for about 10 minutes. The pH of the reaction was confirmed and the next step performed if ≥ 7. Water was added (0.55 vol), the reaction was cooled to an internal temperature of about 35 °C, seed crystals of Compound (1) were added, and the reaction was stirred for about 10 mins. Water was added (3.3 vol) dropwise within about 30 minutes, the suspension was cooled within 30 minutes to an internal temperature of about 0 °C to 5 °C, and the reaction was stirred for 15 minutes. The solid was collected by filtration and washed three times with water.

### Step 2b

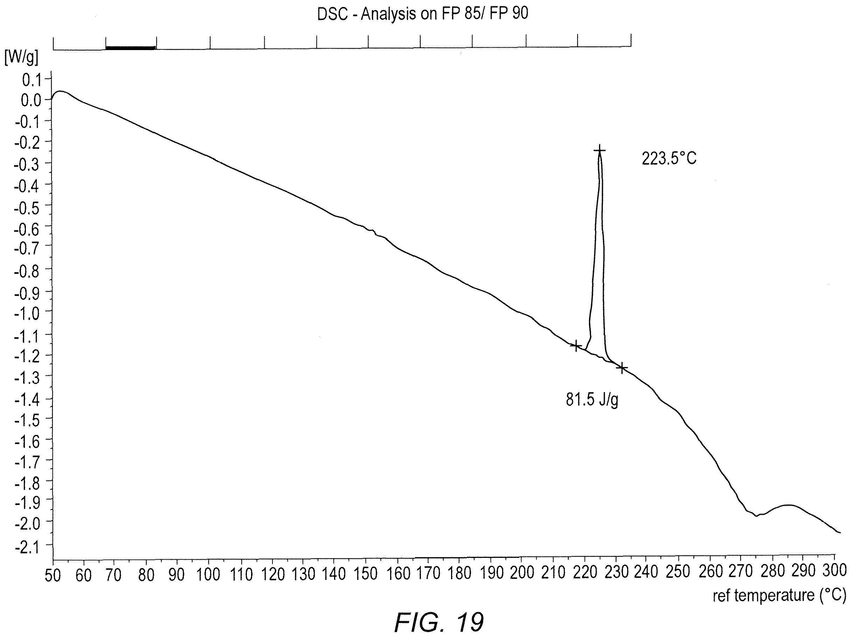

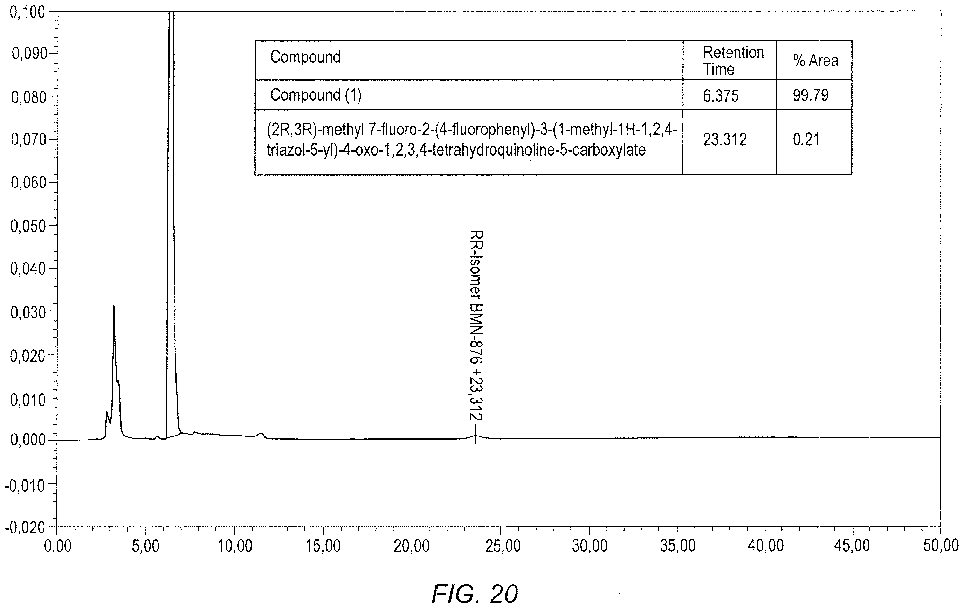

To the product of Step 2a) was added acetone (4 vol), IPA (1 vol), and methanol (1 vol) and the reaction was heated to an internal temperature of about 38 °C to 42 °C resulting in a clear solution. Water (2 vol) and seed crystals of Compound (1) were added and the system was stirred for about 15 minutes at an internal temperature of about 35 °C. Water (342 mL) was added dropwise in about 30 minutes. The suspension was then cooled in 30 min to an internal temperature of about 0 °C to 5 °C and was stirred for an additional 15 minutes. The solid was collected by filtration, washed twice with water, and chiral purity was determined. If ≥ 99% e.e., then the solid was dried at an internal temperature of about 60 °C under reduced pressure to yield Compound (1). The product was characterized by ¹H NMR (Figure 19), ¹³C NMR (Figure 20), IR (Figure 21), DSC (Figure 22), chiral HPLC (Figure 23).

Scheme 2 below describes use of Ac1 10 as a coformer acid for the preparation of Compound (1b) and the chiral resolution of Compound (1).

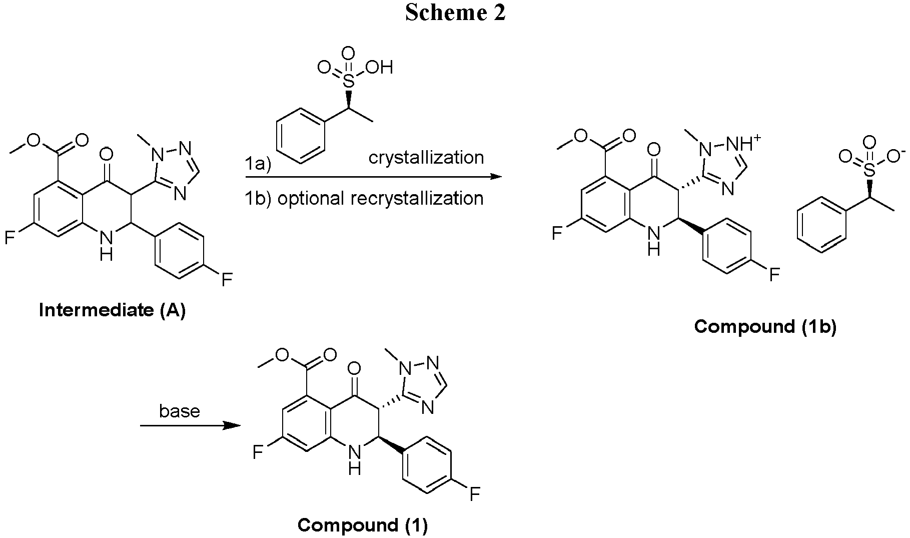

### Example 6 - Preparation of Compound (1) Using Scheme 2 (reference)

### Step 1a

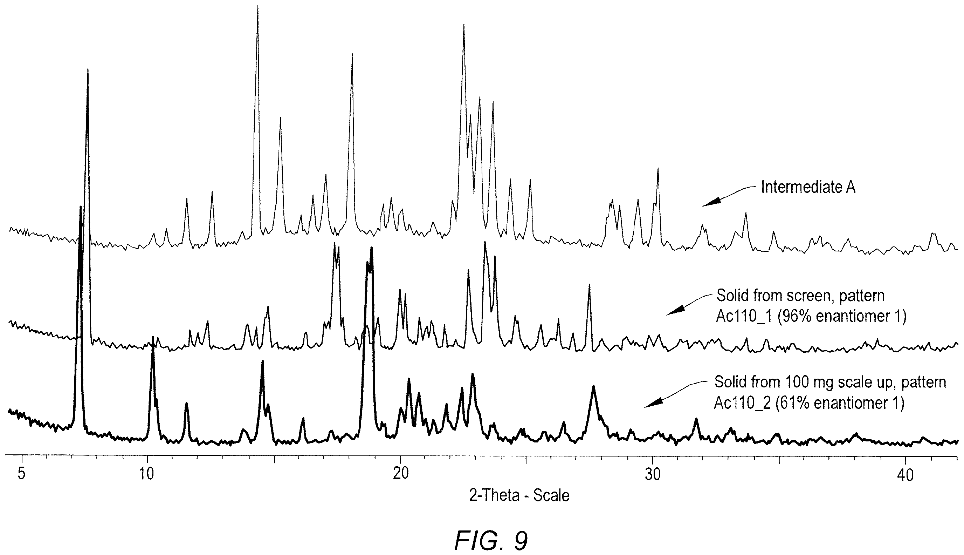

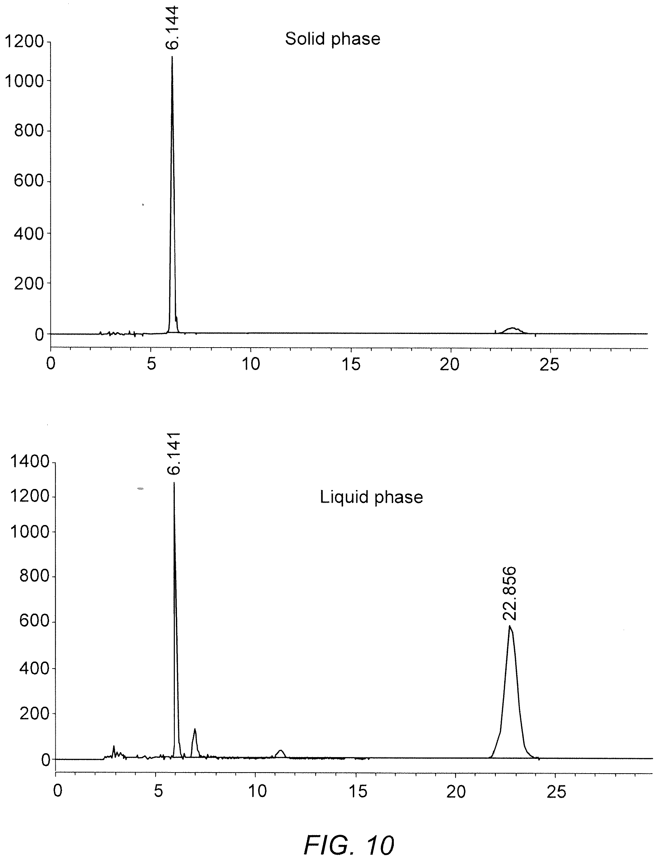

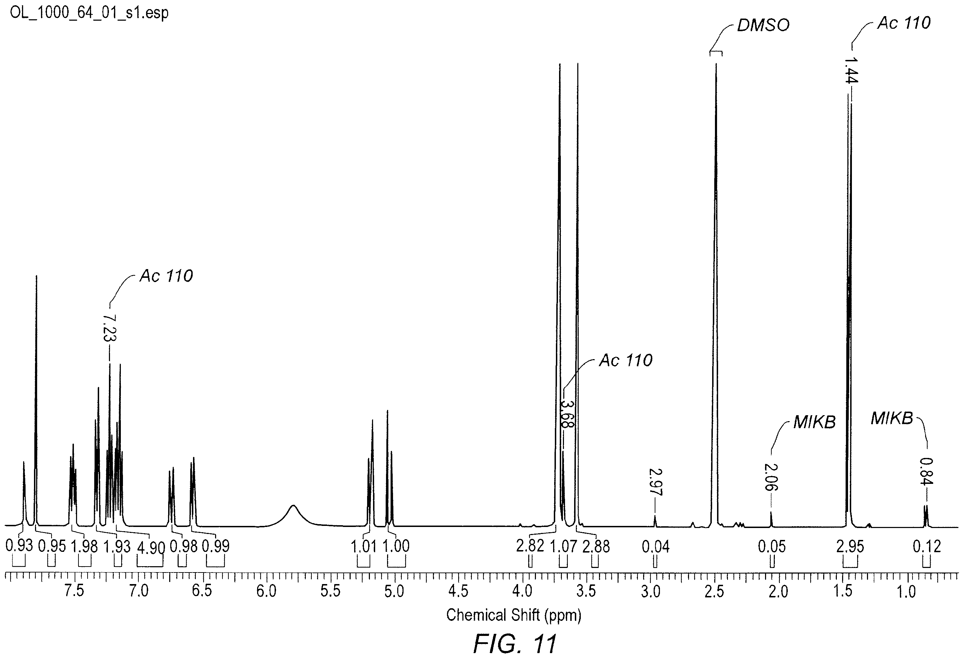

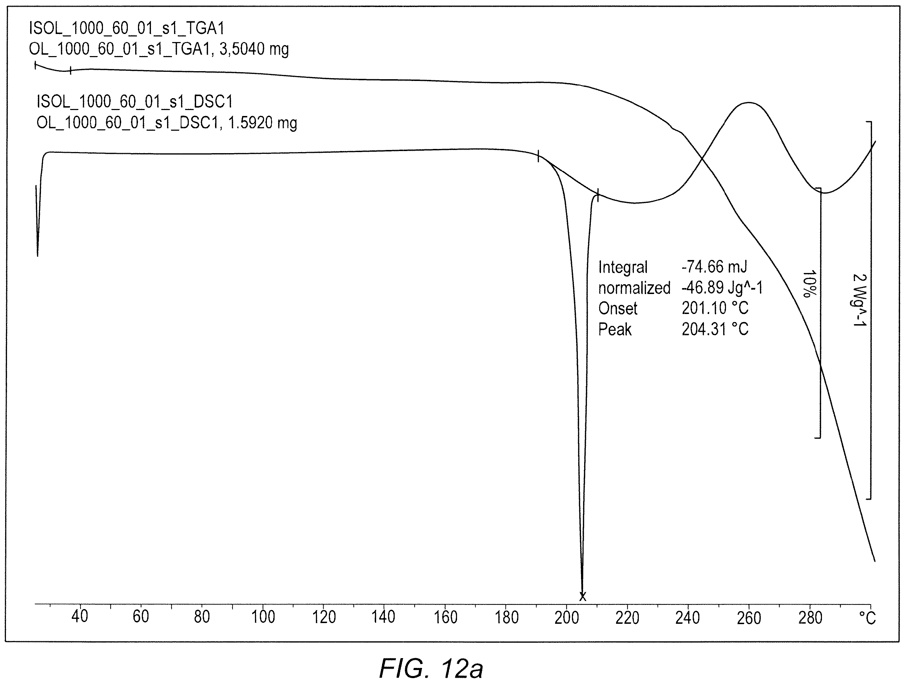

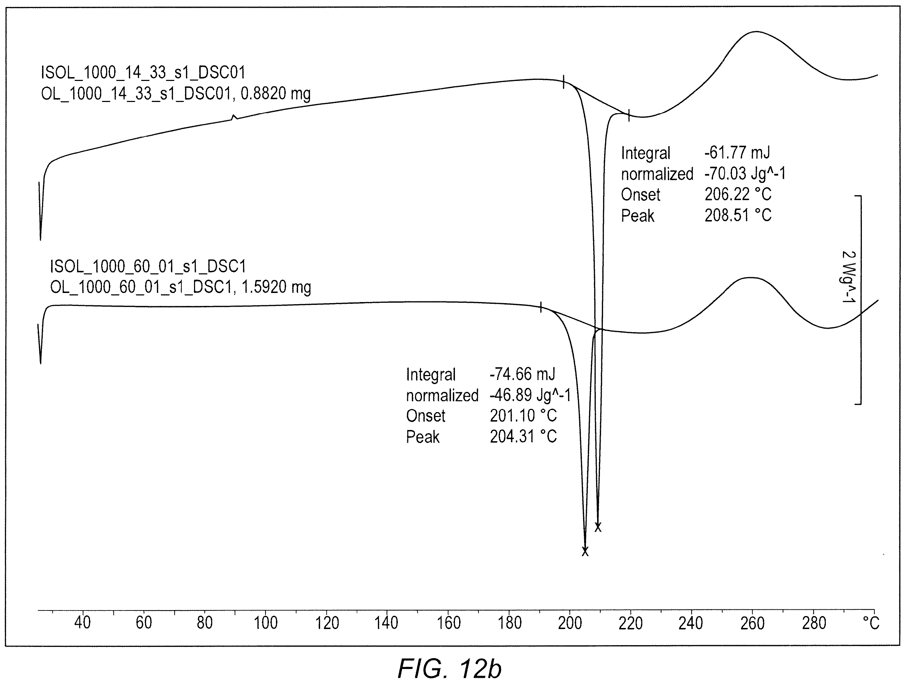

Intermediate (A) (102 mg, 0.256 mmol) was dissolved in MIBK (1 mL, 10 vol.) at 65 °C with stirring. (1*S*)-phenylethanesulfonic acid, prepared using procedures known to one of skill in the art, in MIBK (3.8 M, 80 µL, 1 molar equiv.) was added and a suspension was observed after 30 minutes at 65 °C. The system was kept at 65 °C for another 30 minutes before cooling to 5 °C at 0.1 C/min. After one night at 5 °C, the solid was filtered, dried at 50 °C, 3 mbar pressure for about 2 hours to yield Compound (1b). *See* Figures 9-12 for XRPD (Figure 9), chiral HPLC (Figure 10), ¹H NMR (Figure 11), and TGA/DSC analyses (Figures 12a and 12b). The XRPD diffraction pattern of the solid obtained in Example 5 differed from the XRPD pattern obtained with the solid from in the salt screen of Example 1 and was consistent with the production of different solids in Examples 1 and 5. The ¹H NMR was consistent with the mono-salt with a 0.3% by weight residue of dioxane. In Figure 12a, the thermal behavior was consistent with a non-solvated form exhibiting a melt/degradation at 201 °C. Figure 12b compares the melt pattern of Compound (1b) in Example 5 with Compound (1b) in Example 1.

Steps 1b and 2 can be carried out using procedures similar to those used in Examples 2-5.

### Example 7 - Polymorphism of Compound (1a)

Compound (1) (92% e.e., 10 mg, mmol) was placed in 1.5 mL vials and the solvents (1 mL or less) of Table 3 were added at 50 °C until dissolution was achieved. [(1S)-*endo*]-(+)-3-bromo-10-camphorsulfonic acid was added as a solid at 50 °C. The samples were kept at 50 °C for about 1 hour prior to being cooled to room temperature overnight (uncontrolled cooling rate). Clear solutions were successively cooled to 4 °C, -20 °C and evaporated at room temperature. Any gum obtained after evaporation was re-suspended in diethyl ether. The solid phases generated were characterized by XRPD and if relevant, by ¹H NMR and TGA/DSC.

**Table 3. Compound (1a) Polymorphism Conditions**

| C E: .S. means clear solution and Susp. means suspension. "A" means the XRPD diffraction pattern was new but similar to that for Ac49 in xample 1. "B" means the XRPD diffraction pattern was the same as that for Ac49 in Example 1. "M.E." means molar equiv. | | | | | | | | |
|---|---|---|---|---|---|---|---|---|
| **Solvent** | **Cooled to R.T.** | **C Cooled to 4 °C** | **Cooled to -20 °C** | **Evap. at R.T.** | **Resuspension in diethyl ether** | **XRPD on suspension** | **NMR on suspension** | **Characterization** |
| acetone | C.S. | C.S. | C.S. | Susp. | - | A | 1 equiv. Ac49, 1 M.E. acetone | mono-salt, mono-solvate of acetone |
| MEK | C.S. | C.S. | C.S. | Gum | Gum | - | - | - |
| MIBK | C.S. | C.S. | C.S. | Gum | Gum | - | - | - |
| EtOH | Susp. | - | - | - | - | B | - | ethanolate |
| IPA | Susp. | - | - | - | - | A | 1 equiv Ac49, 0.9 M.E. IPA | mono-salt, mono-solvate of IPA |
| EA | C.S. | C.S. | - | Susp. | - | A | - | Suspected solvate |
| THF | Susp. | - | - | - | - | A | 1 equiv Ac49, 1 M.E. THF | mono-salt, mono-solvate of THF |
| Dioxane | Susp. | - | - | - | - | A | 1 equiv Ac49, 1 M.E. dioxane | mono-salt, mono-solvate of dioxane |
| EtOH 10% in water | C.S. | C.S. | - | Susp. | - | B | - | ethanolate |
| DMF | C.S. | C.S. | - | Gum | Gum | - | - | - |
| Toluene | Susp. | - | - | - | - | free base | - | free base |
| ACN | Susp. | - | - | - | - | A | 1 equiv Ac49, 0.6 M.E. ACN | mono-salt, ACN solvate |
| Heptane | Susp. | - | - | - | - | free base | - | free base |
| Acetone 10% EtOH | Susp. | - | - | - | - | A | 1 equiv Ac49, 0.6 M.E. acetone, 0.2 M.E. EtOH | mixture of solvates or heterosolvate |
| IPA 10% EtOH | Susp. | - | - | - | - | same as for pure IPA | - | mono-salt, mono-solvate of IPA |
| EA 10% EtOH | C.S. | crystals | - | - | - | - | - | heterosolvate |
| THF 10% EtOH | Susp. | - | - | - | - | same as for pure THF | 1 equiv Ac49, 0.7 M.E. THF, 0.2 M.E. EtOH | mono-salt, mono-solvate of IPA |
| Dioxane 10% EtOH | C.S. | C.S. | Frozen solvent | Susp. | | same as for pure dioxane | | mono-salt, mono-solvate of dioxane |
| Toluene 10% EtOH | Susp. | - | - | - | - | A | 1 equiv. Ac49, 0.8 M.E. EtOH | mom-salt, 0.8 equiv ethanolate |
| DMF 10% EtOH | C.S. | C.S. | C.S. | Gum | Gum | - | - | - |

Each of the seven solvents in which solvates were observed (heterosolvates not included) were mixed with MIBK (90% vol). Solutions of Intermediate (A) were prepared in the solvent mixtures (10 vol) at 50 C and [(1*S*)-*endo*]-(+)-3-bromo-10-camphor sulfonic acid (1 molar equivalent) was added. The resulting clear solutions were cooled to 5 °C at 0.2 C/min. Surprisingly, no crystallization was reported in any sample. Seeding was performed with a few crystals of each solvate at about 25 °C. The solid phases were analyzed by XRPD and the liquid phases were analyzed by chiral HPLC. *See* Table 4 for a summary of the results (where "Dias 2" is the (2R, 3R) diastereomer of Compound (1a)) .

**Table 4. Compound (1a) Solvate Analysis**

| **Solvents (1:9)** | **HPLC on the Liquid Phase (% Cmpd (1a))** | **HPLC on the Solid Phase (%** | **XRPD Analysis** |
|---|---|---|---|
| Acetone/MIBK | 25% | 62% | low crystallinity Cmpd. 1a (acetone solvate) + Dias. 2 (non-solvated) |
| IPA/MIBK | 26% | 66% | Cmpd. 1a (IPA solvate) + Dias. 2 (non-solvated) |
| EtOAc/MIBK | 21% | 63% | New pattern + Dias. 2 (non-solvated) |
| THF/MIBK | 18% | 65% | Cmpd. 1a (THF solvate) + Dias. 2 (non-solvated) |
| Dioxane/MIBK | 34% | 65% | Cmpd. 1a (dioxane solvate) + Dias. 2 (non-solvated) |
| ACN/MIBK | 17% | 79% | Cmpd. 1a (ACN solvate) + Dias. 2 (non-solvated) |
| EtOH/MIBK | 9% | 93% | Pure Cmpd. 1a (ethanol solvate) |

As seen in Table 4 above, the ethanol/MIBK system yielded 93% pure Compound (1a) which demonstrates that Compound (1a) does crystallize in a very pure form as an ethanolate solvate.

## Claims

1. A coformer salt of (2*S*,3*S*)-methyl 7-fluoro-2-(4-fluorophenyl)-3-(1-methyl-1*H*-1,2,4-triazol-5-yl)-4-oxo-1,2,3,4-tetrahydroquinoline-5-carboxylate which is [(1*S*)-*endo*]-(+)-3-bromo-10-camphor sulfonic acid salt of (2*S*,3*S*)-methyl 7-fluoro-2-(4-fluorophenyl)-3-(1-methyl-1*H*-1,2,4-triazol-5-yl)-4-oxo-1,2,3,4-tetrahydroquinoline-5-carboxylate, optionally as a solvate and additionally optionally as a hydrate thereof, wherein the coformer salt is in a crystalline form and exhibits solid state ¹³C NMR spectrum with peaks at 210.3, 25.3, 21.8, 20.8, 19.5, and 18.5 ppm ± 0.2 ppm referenced to residual peaks from DMSO-d₆.

2. A method of preparing a coformer salt of (2*S*,3*S*)-methyl 7-fluoro-2-(4-fluorophenyl)-3-(1-methyl-1*H*-1,2,4-triazol-5-yl)-4-oxo-1,2,3,4-tetrahydroquinoline-5-carboxylate according to claim 1 comprising:
(1) treating methyl 7-fluoro-2-(4-fluorophenyl)-3-(1-methyl-1*H*-1,2,4-triazol-5-yl)-4-oxo-1,2,3,4-tetrahydroquinoline-5-carboxylate with a coformer in one or more step la) solvent(s) at an elevated temperature to form a step la) solution;
wherein the step la) solvent(s) are selected from C₁₋₆ ketone, C₁₋₆ alcohol, ethyl acetate, tetrahydrofuran, toluene, acetonitrile, heptane, dioxane, and water;
(2) allowing the step la) solution to stand under conditions sufficient to precipitate the coformer salt in a solid form; and
(3) isolating the coformer salt solid form.

3. The method of claim 2, wherein the coformer salt is a [(1*S*)-*endo*]-(+)-3-bromo-10-camphor sulfonate, and the step la) solvent(s) are selected from acetone, methylethylketone, methylisobutylketone, methanol, ethanol, propanol, isopropanol, and butanol.

4. The method of claim 2 or 3, wherein the coformer salt is a [(1*S*)-*endo*]-(+)-3-bromo-10-camphor sulfonate and the step la) solvents are methylisobutylketone, water, and ethanol.

5. The method of claim 2 or 3, wherein the coformer salt is a [(1*S*)-*endo*]-(+)-3-bromo-10-camphor sulfonate and the step la) solvents are methylisobutylketone and ethanol.

6. The method of any of claims 2-5, further comprising recrystallizing or reslurrying the coformer salt in one or more step 1b) solvent(s).

7. The method of any of claims 2-6, further comprising:
(4) suspending the coformer salt of (2*S*,3*S*)-methyl 7-fluoro-2-(4-fluorophenyl)-3-(1-methyl-1*H*-1,2,4-triazol-5-yl)-4-oxo-1,2,3,4-tetrahydroquinoline-5-carboxylate in one or more step 2a) solvent(s) at room temperature or at elevated temperature, to form a step 2a) solution and treating the step 2a) solution with a base selected from NaOH, aqueous NH₃, NaCO₃, NaOAc, or NaHCO₃;
wherein step 2a) solvent(s) are selected from C₁₋₆ ketone, C₁₋₆ alcohol, and water;
(5) allowing the step 2a) solution to stand under conditions sufficient to precipitate a solid form of the co former salt; and
(6) isolating the (2*S*,3*S*)-methyl 7-fluoro-2-(4-fluorophenyl)-3-(1-methyl-1*H*-1,2,4-triazol-5-yl)-4-oxo-1,2,3,4-tetrahydroquinoline-5-carboxylate solid form.

8. The method of any of claims 2-7, wherein the step 2a) solvent(s) are selected from acetone, methylethylketone, methylisobutylketone, methanol, ethanol, propanol, or isopropanol; and the base is aqueous NH₃.

9. The method of any of claims 2-8, wherein the step 2a) solvents are acetone, methanol, and isopropanol; and the base is aqueous NH₃.

10. The method of any of claims 2-9, further comprising recrystallizing or reslurrying the coformer salt in one or more step 2b) solvent(s).

11. A method of preparing (2*S*,3*S*)-methyl 7-fluoro-2-(4-fluorophenyl)-3-(1-methyl-1*H*-1,2,4-triazol-5-yl)-4-oxo-1,2,3,4-tetrahydroquinoline-5-carboxylate optionally as a solvate and additionally optionally as a hydrate, said method comprising treating a coformer salt of (2*S*,3*S*)-methyl 7-fluoro-2-(4-fluorophenyl)-3-(1-methyl-1*H*-1,2,4-triazol-5-yl)-4-oxo-1,2,3,4-tetrahydroquinoline-5-carboxylate as defined in claim 1 with a base and isolating the (2*S*,3*S*)-methyl 7-fluoro-2-(4-fluorophenyl)-3-(1-methyl-1*H*-1,2,4-triazol-5-yl)-4-oxo-1,2,3,4-tetrahydroquinoline-5-carboxylate.

## Patentansprüche

1. Coformersalz von (2*S*,3*S*)-Methyl-7-fluor-2-(4-fluorphenyl)-3-(1-methyl-1*H*-1,2,4-triazol-5-yl)-4-oxo-1,2,3,4-tetrahydrochinolin-5-carboxylat, das [(1*S*)-*endo*]-(+)-3-Brom-10-camphersulfonsäuresalz von (2*S*,3*S*)-Methyl-7-fluor-2-(4-fluorphenyl)-3-(1-methyl-1*H*-1,2,4-triazol-5-yl)-4-oxo-1,2,3,4-tetrahydrochinolin-5-carboxylat, gegebenenfalls als ein Solvat und zusätzlich gegebenenfalls als ein Hydrat davon, wobei das Coformersalz in kristalliner Form vorliegt und ein ¹³C-Festkörper-NMR-Spektrum mit Peaks bei 210,3, 25,3, 21,8, 20,8, 19,5 und 18,5 ppm ± 0,2 ppm, bezogen auf Restpeaks von DMSO-d₆, aufweist.

2. Verfahren zur Herstellung eines Coformersalzes von (2*S*,3*S*)-Methyl-7-fluor-2-(4-fluorphenyl)-3-(1-methyl-1*H-*1,2,4-triazol-5-yl)-4-oxo-1,2,3,4-tetrahydrochinolin-5-carboxylat nach Anspruch 1, umfassend:
(1) Behandeln von Methyl-7-fluor-2-(4-fluorphenyl)-3-(1-methyl-1*H*-1,2,4-triazol-5-yl)-4-oxo-1,2,3,4-tetrahydrochinolin-5-carboxylat mit einem Coformer in einem oder mehreren Lösungsmittel(n) von Schritt 1a) bei erhöhter Temperatur, um eine Lösung von Schritt 1a) zu bilden;
wobei das/die Lösungsmittel von Schritt 1a) aus C₁₋₆-Keton, C₁₋₆-Alkohol, Ethylacetat, Tetrahydrofuran, Toluol, Acetonitril, Heptan, Dioxan und Wasser ausgewählt ist/sind;
(2) Stehenlassen der Lösung aus Schritt 1a) unter Bedingungen, die ausreichen, um das Coformersalz in fester Form auszufällen; und
(3) Isolierung der festen Form des Coformersalzes.

3. Verfahren nach Anspruch 2, wobei das Coformersalz ein [(1S)-endo]-(+)-3-Brom-10-camphersulfonat ist und das/die Lösungsmittel von Schritt 1a) aus Aceton, Methylethylketon, Methylisobutylketon, Methanol, Ethanol, Propanol, Isopropanol und Butanol ausgewählt ist/sind.

4. Verfahren nach Anspruch 2 oder 3, wobei das Coformersalz ein [(1*S*)-*endo*]-(+)-3-Brom-10-camphersulfonat ist und die Lösungsmittel von Schritt 1a) Methylisobutylketon, Wasser und Ethanol sind.

5. Verfahren nach Anspruch 2 oder 3, wobei das Coformersalz ein [(1*S*)-*endo*]-(+)-3-Brom-10-camphersulfonat ist und die Lösungsmittel von Schritt 1a) Methylisobutylketon und Ethanol sind.

6. Verfahren nach einem der Ansprüche 2 bis 5, umfassend ferner das Umkristallisieren oder Wiederaufschlämmen des Coformersalzes in einem oder mehreren Lösungsmittel(n) von Schritt 1b).

7. Verfahren nach einem der Ansprüche 2 bis 6, das ferner Folgendes umfasst:
(4) Suspendieren des Coformersalzes von (2*S*,3*S*)-Methyl-7-fluor-2-(4-fluorphenyl)-3-(1-methyl-1*H*-1,2,4-triazol-5-yl)-4-oxo-1,2,3,4-tetrahydrochinolin-5-carboxylat in einem oder mehreren Lösungsmittel(n) von Schritt 2a) bei Raumtemperatur oder bei erhöhter Temperatur, um eine Lösung von Schritt 2a) zu bilden, und Behandeln der Lösung von Schritt 2a) mit einer Base, ausgewählt aus NaOH, wässrigem NH₃, NaCO₃, NaOAc oder NaHCO₃;
wobei das/die Lösungsmittel von Schritt 2a) aus C₁₋₆-Keton, C₁₋₆-Alkohol und Wasser ausgewählt ist/sind;
(5) Stehenlassen der Lösung von Schritt 2a) unter Bedingungen, die ausreichen, um eine feste Form des Coformersalzes auszufällen; und
(6) Isolierung der festen Form von (2S,3S)-Methyl-7-fluor-2-(4-fluorphenyl)-3-(1-methyl-1H-1,2,4-triazol-5-yl)-4-oxo-1,2,3,4-tetrahydrochinolin-5-carboxylat.

8. Verfahren nach einem der Ansprüche 2-7, wobei das/die Lösungsmittel von Schritt 2a) aus Aceton, Methylethylketon, Methylisobutylketon, Methanol, Ethanol, Propanol oder Isopropanol ausgewählt ist/sind und die Base wässriges NH₃ ist.

9. Verfahren nach einem der Ansprüche 2-8, wobei die Lösungsmittel von Schritt 2a) Aceton, Methanol und Isopropanol sind und die Base wässriges NH₃ ist.

10. Verfahren nach einem der Ansprüche 2 bis 9, umfassend ferner das Umkristallisieren oder Wiederaufschlämmen des Coformersalzes in einem oder mehreren Lösungsmittel(n) von Schritt 2b).

11. Verfahren zur Herstellung von (2S,3S)-Methyl-7-fluor-2-(4-fluorphenyl)-3-(1-methyl-1*H*-1,2,4-triazol-5-yl)-4-oxo-1,2,3,4-tetrahydrochinolin-5-carboxylat, gegebenenfalls als ein Solvat und zusätzlich gegebenenfalls als ein Hydrat, wobei das Verfahren die Behandlung eines Coformersalzes von (2*S*,3*S*)-Methyl-7-fluor-2-(4-fluorphenyl)-3-(1-methyl-1*H-*1,2,4-triazol-5-yl)-4-oxo-1,2,3,4-tetrahydrochinolin-5-carboxylat, wie in Anspruch 1 definiert, mit einer Base und Isolieren des (2*S*,3*S*)-Methyl-7-fluoro-2-(4-fluorophenyl)-3-(1-methyl-1*H*-1,2,4-triazol-5-yl)-4-oxo-1,2,3,4-tetrahydrochinolin-5-carboxylats umfasst.

## Revendications

1. Sel coformeur de 7-fluoro-2-(4-fluorophényl)-3-(1-méthyl-1H-1,2,4-triazol-5-yl)-4-oxo-1,2,3,4-tétrahydroquinoléine-5-carboxylate de (2*S*,3*S*)-méthyle qui est un sel d'acide [(1*S*)-*endo*]-(+)-3-bromo-10-camphresulfonique de 7-fluoro-2-(4-fluorophényl)-3-(1-méthyl-1*H*-1,2,4-triazol-5-yl)-4-oxo-1,2,3,4-tétrahydroquinoléine-5-carboxylate de (2*S*,3*S*)-méthyle, optionnellement sous la forme d'un solvate et en outre optionnellement sous la forme d'un hydrate de celui-ci, dans lequel le sel coformeur est sous une forme cristalline et présente un spectre RMN ¹³C à l'état solide avec des pics à 210,3, 25,3, 21,8, 20,8, 19,5, et 18,5 ppm ± 0,2 ppm avec pour référence les pics résiduels du DMSO-d₆.

2. Procédé de préparation d'un sel coformeur de 7-fluoro-2-(4-fluorophényl)-3-(1-méthyl-1*H*-1,2,4-triazol-5-yl)-4-oxo-1,2,3,4-tétrahydroquinoléine-5-carboxylate de (2*S*,3*S*)-méthyle selon la revendication 1 comprenant :
(1) le traitement de 7-fluoro-2-(4-fluorophényl)-3-(1-méthyl-1*H*-1,2,4-triazol-5-yl)-4-oxo-1,2,3,4-tétrahydroquinoléine-5-carboxylate de méthyle avec un coformeur dans un ou plusieurs solvant(s) de l'étape 1a) à une température élevée pour former une solution de l'étape 1a) ;
dans lequel le(s) solvant(s) de l'étape 1a) sont sélectionnés parmi une cétone en C₁₋₆, un alcool en C₁₋₆, l'acétate d'éthyle, le tétrahydrofurane, le toluène, l'acétonitrile, l'heptane, le dioxane, et l'eau ;
(2) le fait de laisser la solution de l'étape 1a) reposer dans des conditions suffisantes pour que le sel coformeur précipite sous une forme solide ; et
(3) l'isolement de la forme solide du sel coformeur.

3. Procédé selon la revendication 2, dans lequel le sel coformeur est un [(1*S*)-*endo*]-(+)-3-bromo-10-camphre sulfonate, et le(s) solvant(s) de l'étape 1a) sont sélectionnés parmi l'acétone, la méthyléthylcétone, la méthylisobutylcétone, le méthanol, l'éthanol, le propanol, l'isopropanol, et le butanol.

4. Procédé selon la revendication 2 ou 3, dans lequel le sel coformeur est un [(1*S*)-*endo*]-(+)-3-bromo-10-camphre sulfonate, et les solvants de l'étape 1a) sont la méthylisobutylcétone, l'eau, et l'éthanol.

5. Procédé selon la revendication 2 ou 3, dans lequel le sel coformeur est un [(1*S*)-*endo*]-(+)-3-bromo-10-camphre sulfonate et les solvants de l'étape 1a) sont la méthylisobutylcétone et l'éthanol.

6. Procédé selon l'une des revendications 2 à 5, comprenant en outre la recristallisation ou la remise en suspension du sel coformeur dans un ou plusieurs solvant(s) de l'étape 1b).

7. Procédé selon l'une des revendications 2 à 6, comprenant en outre :
(4) la mise en suspension du sel coformeur de 7-fluoro-2-(4-fluorophényl)-3-(1-méthyl-1*H*-1,2,4-triazol-5-yl)-4-oxo-1,2,3,4-tétrahydroquinoléine-5-carboxylate de (2*S*,3*S*)-méthyle dans un ou plusieurs solvant(s) de l'étape 2a) à température ambiante ou à une température élevée, pour former une solution de l'étape 2a) et le traitement de la solution de l'étape 2a) avec une base sélectionnée parmi le NaOH, le NH₃ aqueux, le NaCO₃, le NaOAc, ou le NaHCO₃ ;
dans lequel le(s) solvant(s) de l'étape 2a) sont sélectionnés parmi une cétone en C₁₋₆, un alcool en C₁₋₆, et de l'eau ;
(5) le fait de laisser la solution de l'étape 2a) reposer dans des conditions suffisantes pour précipiter une forme solide du sel coformeur ; et
(6) l'isolement de la forme solide du 7-fluoro-2-(4-fluorophényl)-3-(1-méthyl-1*H*-1,2,4-triazol-5-yl)-4-oxo-1,2,3,4-tétrahydroquinoléine-5-carboxylate de (2*S*,3*S*)-méthyle.

8. Procédé selon l'une des revendications 2 à 7, dans lequel le(s) solvant(s) de l'étape 2a) sont sélectionnés parmi l'acétone, la méthyléthylcétone, la méthylisobutylcétone, le méthanol, l'éthanol, le propanol, ou l'isopropanol ; et la base est le NH₃ aqueux.

9. Procédé selon l'une des revendications 2 à 8, dans lequel les solvants de l'étape 2a) sont l'acétone, le méthanol, et l'isopropanol ; et la base est le NH₃ aqueux.

10. Procédé selon l'une des revendications 2 à 9, comprenant en outre la recristallisation ou la remise en suspension du sel coformeur dans un ou plusieurs solvant(s) de l'étape 2b).

11. Procédé de préparation de 7-fluoro-2-(4-fluorophényl)-3-(1-méthyl-1*H*-1,2,4-triazol-5-yl)-4-oxo-1,2,3,4-tétrahydroquinoléine-5-carboxylate de (2*S*,3*S*)-méthyle optionnellement sous la forme d'un solvate et en outre optionnellement sous la forme d'un hydrate, ledit procédé comprenant le traitement d'un sel coformeur de 7-fluoro-2-(4-fluorophényl)-3-(1-méthyl-1*H*-1,2,4-triazol-5-yl)-4-oxo-1,2,3,4-tétrahydroquinoléine-5-carboxylate de (2*S*,3*S*)-méthyle tel que défini dans la revendication 1 avec une base et l'isolement du 7-fluoro-2-(4-fluorophényl)-3-(1-méthyl-1H-1,2,4-triazol-5-yl)-4-oxo-1,2,3,4-tétrahydroquinoléine-5-carboxylate de (2*S*,3*S*)-méthyle.
